# EUROPEAN PATENT APPLICATION

(11) **EP 3 786 232 A1**
(43) Date of publication of application: **03.03.2021**
(21) Application number: 19792001.0
(22) Date of filing: 26.04.2019
(51) Int. Cl.: C08L 101/00, C08F 2/22, C08F 251/02, C08L 1/02, C08B 5/00, C08B 11/12, C08B 15/04, C08L 101/16

(54) **COMPOSITE PARTICLES, METHOD FOR PRODUCING COMPOSITE PARTICLES, DRY POWDER, COMPOSITION FOR APPLICATION TO SKIN, AND METHOD FOR PRODUCING COMPOSITION FOR APPLICATION TO SKIN**

(30) Priority: 27.04.2018 JP 2018087500; 12.10.2018 JP 2018193407
(71) Applicant: Toppan Printing Co., Ltd., Tokyo 110-0016 (JP)
(72) Inventor: YABUHARA Yasushi, Tokyo 110-0016 (JP); ISOGAI Takuya, Tokyo 110-0016 (JP)
(74) Representative: Hoffmann Eitle
(86) International application number: PCT/JP2019/018047
(87) International publication number: WO 2019/208802

(57) **Abstract**

Provided are novel composite particles that are biodegradable and easy to handle while maintaining the characteristics of cellulose nanofibers, a method of producing composite particles, a dry powder containing the composite particles, and a skin application composition and a method of producing the skin application composition. For this purpose, for example, a composite particle (5) contains at least one type of particle (3) and fine cellulose (1) with which at least part of a surface of the particle (3) is coated, wherein the particle (3) and the fine cellulose (1) are inseparable.

## Description

### [Technical Field]

The present invention relates to composite particles of fine cellulose and a biodegradable compound, a method of producing composite particles of fine cellulose and a biodegradable compound, a dry powder containing the composite particles, a skin application composition containing composite particles of fine cellulose and a polymer, and a method of producing the skin application composition.

### [Background Art]

Skin application products such as personal care products intended to be used for skin care, makeup, or the like contain a composition obtained by adding various functional materials to a dispersal solvent containing water, alcohol, oil, and the like at a given combination ratio. These functional additives are used to satisfy various required characteristics such as moisture retention properties, dispersion stability, shape retention properties after application to the skin or the like, and a better feeling of the composition when applied to the skin.

Meanwhile, in recent years, attempts have been actively made to utilize, as a novel functional material, fine cellulose fibers obtained by finely grinding cellulose fibers in wood until at least one dimension of a structure of the cellulose has a nanometer-order size.

For example, Patent Literature 1 discloses repeated mechanical treatment using a blender or a grinder applied to wood cellulose provides fine cellulose fibers, that is, cellulose nanofibers (hereinafter also referred to as CNF). The CNF obtained by this method has been reported to have a minor axis diameter in the range of 10 to 50 nm and a major axis diameter in the range of 1 µm to 10 mm. The CNF has a strength 5 or more times that of steel, with 1/5 the weight of steel, and has an enormous specific surface area of 250 m²/g or more. Thus, the CNF is expected to be used as a resin reinforcement filler or an adsorbent.

Furthermore, attempts have been actively made to produce CNF by chemically treating cellulose fibers in wood in advance to facilitate fine grinding, followed by fine grinding by low-energy mechanical treatment such as treatment using a household blender. The chemical treatment method is not particularly limited, but is preferably a method in which an anionic functional group is introduced into the cellulose fibers to facilitate fine grinding. When an anionic functional group is introduced into the cellulose fibers, a solvent is more likely to enter a cellulose microfibril structure due to the osmotic pressure effect, leading to significant reduction in energy required to finely grind the cellulose raw material. The method of introducing an anionic functional group is not particularly limited, and for example, Non Patent Literature 1 discloses a method in which a surface of fine fibers of cellulose is subjected to selective phosphoric acid esterification. Patent Literature 2 discloses a method in which cellulose is allowed to react with monochloroacetic acid or sodium monochloroacetate in a high-concentration alkaline aqueous solution so as to be carboxymethylated. Alternatively, to introduce a carboxyl group, the cellulose may be allowed to directly react with a carboxylic acid anhydride compound such as maleic acid or phthalic acid gasified in an autoclave.

A method has been reported in which 2,2,6,6-tetramethylpiperidinyl-1-oxy radical (TEMPO), which is a relatively stable N-oxyl compound, is used as a catalyst to selectively oxidize a surface of fine fibers of cellulose (see, for example, Patent Literature 3). An oxidation reaction using TEMPO as a catalyst (TEMPO oxidation reaction) enables an environmentally-friendly chemical modification that proceeds in a water system at normal temperature under normal pressure. When the TEMPO oxidation reaction is applied to cellulose in wood, the reaction does not proceed inside crystals of the cellulose, and an alcoholic primary carbon of a cellulose molecular chain on a crystal surface of the cellulose can be selectively converted into a carboxyl group.

As a result of the osmotic pressure effect due to ionization of the carboxyl groups selectively introduced to the crystal surface by the TEMPO oxidation, cellulose single nanofibers (hereinafter also referred to as CSNF) can be obtained which are cellulose microfibrils dispersed in a solvent. The CSNF exhibits high dispersion stability derived from the carboxyl groups on the surface. CSNF derived from wood that is obtained from wood by the TEMPO oxidation reaction is a structure having a high aspect ratio with a minor axis diameter of approximately 3 nm and a major axis diameter in the range of several tens of nanometers to several micrometers. A CSNF water dispersion liquid and an object formed of CSNF have been reported to have high transparency. Patent Literature 4 reports that a laminate film obtained by application and drying of a CSNF dispersion liquid has gas barrier properties.

As an example of a composition for a skin application product containing fine cellulose, for example, Patent Literature 6 discloses a skin external preparation containing TEMPO-oxidized cellulose nanofibers, and reports that the skin external preparation has high moisture retention properties and a good feeling in use.

For practical application of CNF, there is a problem that a CNF dispersion liquid obtained from CNF has a low solid concentration in the range of approximately 0.1 to 5%. For example, transporting a fine cellulose dispersion means transporting a large amount of solvent, thereby causing problems such as higher transport cost and significant deterioration in business efficiency. Furthermore, the use of CNF as a resin reinforcement additive causes a problem of lower addition efficiency due to the low solid content and a problem that when water serving as a solvent is immiscible with resin, the formation of a complex is difficult. Furthermore, when CNF is handled in a hydrous state, due to the risk of spoilage of a hydrous CNF dispersion, measures such as refrigeration storage and preservative treatment are required, which may increase cost. Furthermore, the use of CNF as a cosmetics composition as described above also causes a problem of lower addition efficiency due to the low solid content.

In cosmetics, CNF is also expected to be used without being dispersed in a solvent, such as in the form of a powder foundation with no water or with a low water content.

However, if a solvent of a fine cellulose dispersion liquid is removed simply by heat drying or the like, pieces of fine cellulose are aggregated and keratinized or formed into a film, and this makes it difficult for the CNF to stably exhibit its function as an additive. Furthermore, due to the low solid concentration of the CNF, the step of removing the solvent by drying requires enormous energy, which may cause deterioration in business business efficiency.

Thus, since the handling of CNF in the form of a dispersion liquid causes deterioration in business efficiency, provision of a novel handling state that allows easy handling of CNF is strongly desired.

As described above as examples, various studies have been performed to develop high-performance members that impart new functionality to fine cellulose such as CNF and CSNF, which are carbon neutral materials.

As described above, CNF in the form of a dispersion liquid causes an excessive solvent, resulting in significant deterioration in business efficiency when the CNF is used as a composition for a skin application product. That is, provision of a novel handling state that allows easy handling of CNF while maintaining the characteristics of the CNF is strongly desired.

Meanwhile, as a functional material in various fields (e.g., skin application products), various microparticles and microcapsules have been conventionally used in practical application. Microparticles are typically microsized particles formed of various polymers such as polyethylene and polypropylene, and are used, for example, as a filler, a spacer, an abrasive, and the like. In addition, microparticles are widely used for materials applied to the skin such as a base material of a foundation and a keratin removing agent for a facial cleanser. Examples of a conventional method of producing a resin powder include a method in which a monomer emulsified by using a surfactant is polymerized to produce a resin powder and a method in which, while a lump of resin is being cooled to a low temperature in the range of -50 to -180°C, the lump of resin is mechanically ground and classified to obtain a fine powder (Patent Literature 5). However, the former causes, for example, foaming problems at steps such as stirring, transfer, addition of auxiliary agents, and coating, leading to reduction in production efficiency, and also has a problem of effluent load due to leakage of the surfactant from the system. The latter has a problem such as considerable time being required for grinding and classification, and the power having a non-uniform shape.

Furthermore, attempts have been made to impart and exhibit further functionality of microparticles by forming a microcapsule structure in which a surface of the microparticles serving as a core material is coated with a wall film. Specifically, for example, by incorporating, into the core material, a functional material such as a magnetic material, a pharmaceutical product, an agricultural chemical, a fragrance, an adhesive, an enzyme, a pigment, or a dye and then microencapsulating the material, it is possible to protect the functional material and control release behavior. A functional material may be added to the wall film with which the core material is coated. In recent years, an environmentally-friendly biodegradable powder that can be decomposed by microorganisms in nature after being used and can be finally converted into water and carbon dioxide is strongly desired.

Thus, provision of a novel handling state that allows easy handling of biodegradable microparticles is strongly desired.

Due to a high specific surface area of microparticles with a micro size, typically, the microparticles are easily aggregated, leading to low dispersion stability. Therefore, provision of novel microparticles that have good dispersion stability and are applicable to skin application products is also strongly desired.

### [Citation List]

### [Patent Literature]

[PTL 1] JP 2010-216021 A
[PTL 2] WO 2014/088072 A
[PTL 3] JP 2008-001728 A
[PTL 4] WO 2013/042654 A
[PTL 5] JP 2001/288273 A
[PTL 6] JP 2017-109946 A

### [Non Patent Literature]

[NPL 1] Noguchi Y, Homma I, Matsubara Y. Complete nanofibrillation of cellulose prepa red by phosphorylation. Cellulose. 2017; 24: 1295.10.1007/s10570-017-1191-3

### [Summary of the Invention]

### [Technical Problem]

The present invention has been made in view of such circumstances, and has an object of providing novel composite particles that are biodegradable and easy to handle while maintaining characteristics of cellulose nanofibers, a method of producing the composite particles, and a dry powder containing the composite particles.

Furthermore, the present invention has an object of solving the problem of having excessive solvent of cellulose nanofibers, and providing a skin application composition containing microparticles in a novel state that can be produced by a simple method.

### [Solution to Problem]

In order to solve the above problem, the present invention proposes the following means.

A composite particle according to an aspect of the present invention includes: a particle containing at least one type of biodegradable compound; and fine cellulose with which at least part of a surface of the particle containing the biodegradable compound is coated, wherein the particle containing the biodegradable compound and the fine cellulose are inseparable.

A method of producing a composite particle according to an aspect of the present invention includes: a first step of defibrating a cellulose raw material in a solvent to obtain a dispersion liquid of fine cellulose; a second step of coating at least part of a surface of a droplet containing a biodegradable compound with the fine cellulose in the dispersion liquid to stabilize the droplet containing the biodegradable compound as an emulsion; and a third step of, while at least part of the surface of the droplet containing the biodegradable compound is coated with the fine cellulose, solidifying the droplet containing the biodegradable compound to form a polymer particle so that at least part of a surface of the polymer particle is coated with the fine cellulose and the polymer particle and the fine cellulose are inseparable.

In the method of producing a composite particle according to an aspect of the present invention, the second step may be one of a step in which a mixture of the biodegradable compound and a polymerizable monomer containing an initiator is added to the dispersion liquid of the fine cellulose and emulsified, a step in which a solution obtained by dissolving the biodegradable compound in a solvent having low immiscibility with the dispersion liquid of the fine cellulose is added to the dispersion liquid of the fine cellulose and emulsified, and a step in which the biodegradable compound that is solid at normal temperature is added to the dispersion liquid of the fine cellulose and heated to a melting point of the biodegradable compound or more so that the biodegradable compound is melted and emulsified.

A dry powder according to an aspect of the present invention includes the composite particle described above and has a solid content ratio of 80% or more.

A skin application composition according to another aspect of the present invention is characterized by including a composite particle that contains at least one type of polymer particle and has a coating layer that is composed of fine cellulose and provided on a surface of the polymer particle, wherein the polymer particle and the fine cellulose are bonded to each other and inseparable.

A method of producing a skin application composition according to the present invention includes: a first step of defibrating a cellulose raw material in a solvent to obtain a dispersion liquid of fine cellulose; a second step of coating a surface of a polymer droplet with the fine cellulose in the dispersion liquid to stabilize the polymer droplet as an emulsion; and a third step of solidifying the polymer droplet to obtain a composite particle in which a polymer is coated with the fine cellulose.

### [Advantageous Effects of the Invention]

An aspect of the present invention provides novel composite particles that are biodegradable and easy to handle while maintaining the characteristics of cellulose nanofibers, a method of producing the composite particles, and a dry powder containing the composite particles.

Furthermore, another aspect of the present invention provides a novel handling state that allows easy handling of CNF while maintaining the characteristics of the CNF, and novel microparticles that have good dispersion stability and are applicable to skin application products.

### [Brief Description of the Drawings]

Fig. 1 is a schematic diagram of an O/W Pickering emulsion containing CNF according to an embodiment of the present invention, and composite particles obtained by solidifying a biodegradable compound (polymerizable monomer droplets and/or polymer droplets) in the emulsion.
Fig. 2 shows the result of transmission spectrum measurement of a water dispersion liquid of fine cellulose obtained in Examples 1 and 9.
Fig. 3 shows the result of steady-state viscoelasticity measurement, using a rheometer, of the water dispersion liquid of the fine cellulose obtained in Examples 1 and 9.
Fig. 4 is a view (SEM image) showing the result of observation of composite particles obtained in Example 1 using a scanning electron microscope (SEM).
Fig. 5 is a view (SEM image) showing the result of observation of the composite particles obtained in Example 1 using a scanning electron microscope (SEM) at high magnification.
Fig. 6 is a view (SEM image) showing the result of observation of composite particles obtained in Example 9 using a scanning electron microscope (SEM).
Fig. 7 is a view (SEM image) showing the result of observation of the composite particles obtained in Example 9 using a scanning electron microscope (SEM) at high magnification.

### [Description of the Embodiments]

Embodiments of the present invention will be described below with reference to the drawings. In the drawings described below, the corresponding portions are given the same reference numerals, and redundant description is omitted as appropriate. The embodiments are merely examples of configurations for embodying the technical idea of the present invention, and do not specify the materials, shapes, structures, arrangements, sizes, or the like of the components. The technical idea of the present invention may be modified in various manners within the technical scope defined by the claims.

### [First embodiment]

### <Composite particle of fine cellulose and biodegradable compound>

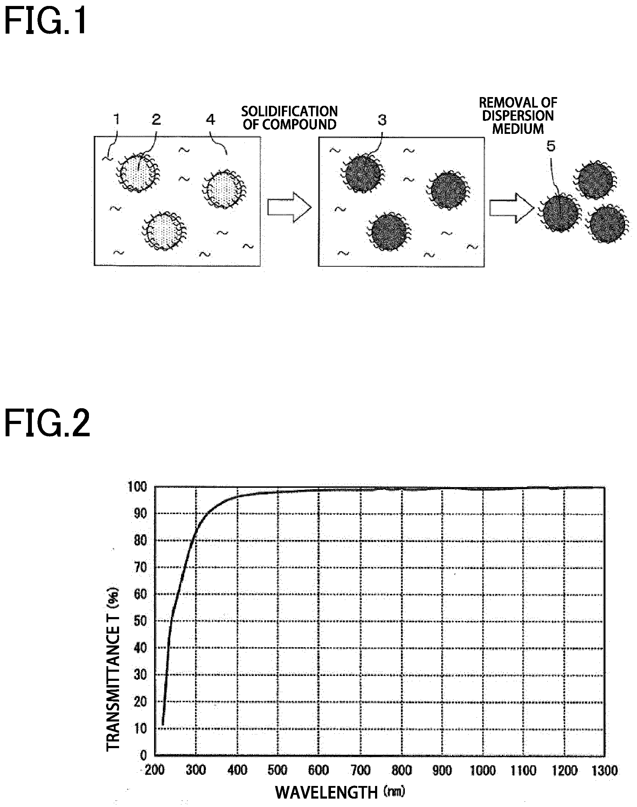

First, a composite particle 5 of fine cellulose and a biodegradable compound particle according to a first embodiment of the present invention will be described. Fig. 1 is a schematic diagram of an O/W Pickering emulsion containing cellulose nanofibers (hereinafter also referred to as CNF or cellulose) 1, and the composite particle 5 obtained by solidifying a biodegradable compound in the emulsion. The term "fine cellulose" means fibrous cellulose having a number average minor axis diameter in the range of 1 nm or more and 1000 nm or less. The term "biodegradable" means properties of decomposing and being eliminated in global environments such as soil or seawater. In general, in soil or seawater, a substance such as a polymer is eliminated by decomposition by enzymes of microorganisms, and in vivo, such a substance is eliminated by decomposition by physicochemical hydrolysis with no need for enzymes.

The composite particle 5 is a composite particle that contains a compound particle 3, which is a particle containing at least one type of biodegradable compound, and that has a coating layer composed of a fine cellulose 1 and provided on a surface of the biodegradable compound particle 3 and in which the biodegradable compound particle 3 and the fine cellulose 1 are bonded to each other and are inseparable.

As shown in Fig. 1, the fine cellulose 1 is adsorbed on an interface of compound droplets 2, which are droplets containing a biodegradable compound and dispersed in a dispersion liquid 4, to stabilize the O/W Pickering emulsion. While the stabilized state of the O/W Pickering emulsion is maintained, the biodegradable compound in the emulsion is solidified to produce the composite particles 5 by using the emulsion as a template.

The term "inseparable" means a state in which, for example, even after repetition of an operation of centrifuging a dispersion liquid containing the composite particles 5 and removing the supernatant liquid, followed by addition of a solvent and redispersion to purify and wash the composite particles 5 or an operation of repeatedly washing the composite particles 5 with a solvent by filtration washing using a membrane filter, the fine cellulose 1 and the biodegradable compound particles 3 are not separated and the coating state is maintained in which the biodegradable compound particles 3 are coated with the fine cellulose 1. The coating state can be confirmed, for example, by observing a surface of the composite particle 5 by using a scanning electron microscope. The mechanism of how the fine cellulose 1 is bonded to the biodegradable compound particle 3 in the composite particle 5 is uncertain, but it is expected that, since the composite particle 5 is produced by using, as a template, the O/W emulsion stabilized by the fine cellulose 1, the biodegradable compound droplet 2 in the emulsion is solidified while the fine cellulose 1 is in contact with the biodegradable compound droplet 2, and thus in the composite particle 5 obtained after the solidification, at least part of the fine cellulose 1 on the surface of the biodegradable compound particle 3 serving as a core is incorporated into the biodegradable compound particle 3. Presumably for this reason, the fine cellulose 1 is physically immobilized on the solidified biodegradable compound particle 3, and the biodegradable compound particle 3 and the fine cellulose 1 become inseparable as a result.

The O/W emulsion is also referred to as oil-in-water emulsion, and is an emulsion containing water as a continuous phase in which oil in the form of oil droplets (oil particles) is dispersed.

Since the composite particle 5 is produced by using, as a template, the O/W emulsion stabilized by the fine cellulose 1, the composite particle 5 is characterized by having a spherical shape derived from the O/W emulsion. Specifically, a coating layer composed of the fine cellulose 1 is formed with a relatively uniform thickness on the surface of the spherical biodegradable compound particle 3. The average thickness of the coating layer can be calculated, for example, in the following manner. That is, the composite particle 5 fixed with an embedding resin is cut with a microtome and observed by using a scanning electron microscope. In an image, the thickness of the coating layer in a cross-sectional image of the composite particle 5 is randomly measured at 100 locations, and the average value of the thicknesses is obtained. The composite particle 5 is characterized by being uniformly coated with the coating layer having a relatively uniform thickness. Specifically, the coefficient of variation of the thickness of the coating layer is preferably 0.5 or less, and is more preferably 0.4 or less. If the coefficient of variation of the thickness of the coating layer containing the fine cellulose 1 exceeds 0.5, for example, the composite particle 5 may be difficult to recover.

Furthermore, the fine cellulose 1 preferably has a fiber form derived from a microfibril structure. Specifically, the fine cellulose 1 is preferably fibrous, and has a number average minor axis diameter in the range of 1 nm or more and 1000 nm or less, and a number average major axis diameter of 50 nm or more. The number average major axis diameter is preferably 5 times or more the number average minor axis diameter. In addition, the fine cellulose 1 preferably has a crystal structure of cellulose type I.

### <Method of producing composite particle>

Next, a method of producing a composite particle of the present embodiment will be described. The method of producing a composite particle according to the present embodiment is a method of producing the composite particle 5 including: a step (first step) of defibrating a cellulose raw material in a solvent to obtain the dispersion liquid 4 of the fine cellulose 1; a step (second step) of coating at least part of a surface of the biodegradable compound droplet 2 with the fine cellulose 1 in the dispersion liquid 4 of the fine cellulose 1 to stabilize the biodegradable compound droplet 2 as an emulsion; and a step (third step) of, while at least part of the surface of the biodegradable compound droplet 2 is coated with the fine cellulose 1, solidifying the biodegradable compound droplet 2 to form the biodegradable compound particle 3 so that at least part of a surface of the biodegradable compound particle 3 is coated with the fine cellulose 1 and the biodegradable compound particle 3 and the fine cellulose 1 are inseparable.

The composite particles 5 obtained by the above production method are obtained as a dispersion in the solvent. By removing the solvent, the composite particles 5 are obtained as a dry solid. The method of removing the solvent is not particularly limited, and the composite particles 5 can be obtained as a dry solid, for example, by removing excess moisture by centrifugal separation or filtration, followed by heat drying in an oven. In this case, the dry solid is not formed into a film or an aggregate and is obtained as a fine-grained powder. The reason is uncertain, but it is known that when a solvent is removed from a fine cellulose dispersion, typically, pieces of fine cellulose are strongly aggregated and formed into a film. On the other hand, in the case of the dispersion liquid containing the composite particles 5, since the composite particles 5 are spherical composite particles having a surface on which the fine cellulose 1 is immobilized, even when the solvent is removed, pieces of fine cellulose 1 are not aggregated and the composite particles are only in point contact with each other. This is presumably the reason why the dry solid is obtained as a fine-grained powder. Furthermore, since the composite particles 5 are not aggregated, the composite particles 5 obtained as a dry powder are easily redispersed in a solvent, and after the redispersion, the dispersion liquid also exhibits dispersion stability derived from the fine cellulose 1 bonded to the surface of the composite particles 5.

The dry powder of the composite particles 5 is a dry solid having an advantage of containing little solvent and being redispersible in a solvent. Specifically, the dry powder may have a solid content ratio of 80% or more, 90% or more, or 95% or more. Since most of the solvent can be removed, the dry powder of the composite particles 5 has advantageous effects, for example, in terms of reduction in transport cost, prevention of spoilage, improvement in addition ratio, and improvement in efficiency of kneading with resin. When the dry powder having a solid content ratio of 80% or more is obtained by drying treatment, the fine cellulose 1 easily absorbs moisture, and thus may adsorb moisture in air and cause reduction of the solid content ratio over time. However, considering the technical idea of the present invention having an advantage that the composite particles 5 are easily obtained as a dry powder and the dry powder is redispersible, a dry solid produced by a method of producing a dry solid including a step of causing a dry powder containing the composite particles 5 to have a solid content ratio of 80% or more is defined to be encompassed in the technical scope of the present invention.

The steps will be described below in detail.

### (First step)

The first step is a step of defibrating a cellulose raw material in a solvent to obtain a fine cellulose dispersion liquid. First, a cellulose raw material is dispersed in a solvent to form a suspension. The cellulose raw material is preferably contained in the suspension at a concentration in the range of 0.1% or more and less than 10%. If the cellulose raw material is contained in the suspension at a concentration of less than 0.1%, due to there being an excessive amount of solvent, productivity tends to be deteriorated, which is not preferable. If the cellulose raw material is contained in the suspension at a concentration of 10% or more, due to the defibration of the cellulose raw material, the suspension is rapidly thickened, and thus uniform defibrating treatment tends to be difficult, which is not preferable. The solvent used to produce a suspension preferably contains 50% or more of water. If the ratio of water in the suspension is less than 50%, at the step of defibrating a cellulose raw material in a solvent to obtain a fine cellulose dispersion liquid (described later), dispersion of the fine cellulose 1 tends to be inhibited. A solvent other than water contained in the suspension is preferably a hydrophilic solvent. The hydrophilic solvent is not particularly limited, but is preferably, for example, an alcohol such as methanol, ethanol, and isopropanol; or cyclic ethers such as tetrahydrofuran. If necessary, for example, the pH of the suspension may be adjusted in order to improve dispersibility of the cellulose and the fine cellulose 1 to be generated. Examples of an alkaline aqueous solution used for the pH adjustment include organic alkali such as a sodium hydroxide aqueous solution, a lithium hydroxide aqueous solution, a potassium hydroxide aqueous solution, an ammonia aqueous solution, a tetramethylammonium hydroxide aqueous solution, a tetraethylammonium hydroxide aqueous solution, a tetrabutylammonium hydroxide aqueous solution, and a benzyltrimethylammonium hydroxide aqueous solution. A sodium hydroxide aqueous solution is preferable in terms of cost and the like.

Next, the suspension is subject to physical defibrating treatment to finely grind the cellulose raw material. The physical defibrating treatment is not particularly limited, and may be, for example, mechanical treatment using a high-pressure homogenizer, an ultrahigh-pressure homogenizer, a ball mill, a roll mill, a cutter mill, a planetary mill, a jet mill, an attritor, a grinder, a blender, a homomixer, an ultrasonic homogenizer, a nanogenizer, or aqueous counter collision. By the physical defibrating treatment, the cellulose in the suspension is finely ground to form a dispersion liquid of the cellulose (fine cellulose) 1 finely ground until at least one dimension of a structure of the cellulose has a nanometer-order size. The treatment time and the number of times the physical defibrating treatment is performed can be adjusted to control the number average minor axis diameter and the number average major axis diameter of the fine cellulose 1 to be obtained.

In this manner, a dispersion (fine cellulose dispersion liquid) of the cellulose 1 finely ground until at least one dimension of the structure of the cellulose has a nanometer-order size is obtained. The obtained dispersion can be used as an O/W emulsion stabilizer (described later) as it is or after dilution, concentration, or the like.

In addition to the cellulose and the component used for the pH adjustment, the fine cellulose dispersion may contain an additional component as necessary to such an extent that the effects of the present invention are not impaired. The additional component is not particularly limited, and may be appropriately selected from known additives according to the intended use of the composite particles 5 or the like. Specific examples of the additional component include an organometallic compound such as alkoxysilane or a hydrolysate thereof, an inorganic layered compound, an inorganic needle-like mineral, an antifoaming agent, inorganic particles, organic particles, a lubricant, an antioxidant, an antistatic agent, an ultraviolet absorber, a stabilizer, a magnetic powder, an orientation accelerator, a plasticizer, a crosslinking agent, a magnetic material, a pharmaceutical product, an agricultural chemical, a fragrance, an adhesive, an enzyme, a pigment, a dye, a deodorant, a metal, a metal oxide, and an inorganic oxide.

Since the fine cellulose 1 typically has a fiber form derived from a microfibril structure, the fine cellulose 1 used in the production method of the present embodiment preferably has a fiber form with a size in the following range. That is, the form of the fine cellulose 1 is preferably fibrous. Furthermore, the fibrous fine cellulose 1 preferably has a number average minor axis diameter in the range of 1 nm or more and 1000 nm or less, and more preferably in the range of 2 nm or more and 500 nm or less. If the fine cellulose 1 has a number average minor axis diameter of less than 1 nm, the fine cellulose 1 cannot have a fine cellulose fiber structure that is highly crystalline and rigid, and stabilization of the emulsion and a polymerization reaction using the emulsion as a template tend to be difficult. On the other hand, if the fine cellulose 1 has a number average minor axis diameter of more than 1000 nm, the fine cellulose 1 is excessively large in size for stabilization of the emulsion, and thus control of the size and shape of the composite particles 5 to be obtained tends to be difficult. Furthermore, the number average major axis diameter of the fine cellulose 1 is not particularly limited, but is preferably 5 times or more the number average minor axis diameter. If the fine cellulose 1 has a number average major axis diameter of less than 5 times the number average minor axis diameter, sufficient control of the size and shape of the composite particles 5 tends to be difficult, which is not preferable.

The number average minor axis diameter of the fine cellulose fibers is obtained, for example, by measuring minor axis diameters (minimum diameters) of 100 fibers by transmission electron microscope observation or atomic force microscope observation, and calculating an average value of the minor axis diameters. On the other hand, the number average major axis diameter of the fine cellulose fibers is obtained, for example, by measuring major axis diameters (maximum diameters) of 100 fibers by transmission electron microscope observation or atomic force microscope observation, and calculating an average value of the major axis diameters.

The type and crystal structure of cellulose that can be used as the raw material of the fine cellulose 1 are not particularly limited. Specific examples of a raw material composed of cellulose I type crystals include, in addition to wood-based natural cellulose, non-wood-based natural cellulose such as cotton linters, bamboo, hemp, bagasse, kenaf, bacterial cellulose, hoya cellulose, and valonia cellulose. Furthermore, the raw material of the fine cellulose 1 may also be regenerated cellulose typified by rayon fibers and cuprammonium rayon fibers composed of cellulose II type crystals. Wood-based natural cellulose is preferable as the raw material in terms of ease of material acquisition. The wood-based natural cellulose is not particularly limited, and may be, for example, wood-based natural cellulose typically used to produce cellulose nanofibers, such as softwood pulp, hardwood pulp, or waste paper pulp. Softwood pulp is preferable in terms of ease of purification and fine grinding.

Furthermore, the fine cellulose raw material is preferably chemically modified. More specifically, an anionic functional group is preferably introduced to a crystal surface of the fine cellulose raw material. This is because when an anionic functional group is introduced to the crystal surface of the cellulose, the solvent is more likely to enter between the cellulose crystals due to the osmotic pressure effect, thereby facilitating fine grinding of the cellulose raw material.

The type of anionic functional group introduced to the crystal surface of the cellulose and the introduction method are not particularly limited, but a carboxyl group or a phosphate group is preferable. A carboxyl group is more preferable in terms of ease of selective introduction to the crystal surface of the cellulose.

The method of introducing a carboxyl group to the surface of the cellulose fibers is not particularly limited. Specifically, for example, the cellulose may be allowed to react with monochloroacetic acid or sodium monochloroacetate in a high-concentration alkaline aqueous solution so as to be carboxymethylated. Alternatively, to introduce a carboxyl group, the cellulose may be allowed to directly react with a carboxylic acid anhydride compound such as maleic acid or phthalic acid gasified in an autoclave. Alternatively, a carboxyl group may be introduced by a method in which a co-oxidant is used in the presence of an N-oxyl compound, such as TEMPO, which has high selectivity for oxidation of alcoholic primary carbon, while maintaining the structure as much as possible under relatively mild aqueous conditions. The oxidation using an N-oxyl compound is more preferable in terms of selectivity for a portion to which the carboxyl group is introduced and reduction of the environmental load.

Examples of the N-oxyl compound include TEMPO (2,2,6,6-tetramethylpiperidinyl-1-oxy radical), 2,2,6,6-tetramethyl-4-hydroxypiperidin-1-oxyl, 4-methoxy-2,2,6,6-tetramethylpiperidin-N-oxyl, 4-ethoxy-2,2,6,6-tetramethylpiperidin-N-oxyl, and 4-acetamide-2,2,6,6-tetramethylpiperidin-N-oxyl. Among these, TEMPO which has high reactivity is preferable. The amount of N-oxyl compound to be used is not particularly limited as long as the N-oxyl compound is contained in the amount required for use as a catalyst. Typically, the amount of N-oxyl compound is in the range of approximately 0.01 to 5.0 mass% with respect to the solid content of the wood-based natural cellulose subjected to the oxidation treatment.

The oxidation method using an N-oxyl compound may be, for example, a method in which wood-based natural cellulose is dispersed in water and subjected to oxidation treatment in the presence of the N-oxyl compound. In this method, it is preferable to use a co-oxidant in combination with the N-oxyl compound. In this case, in the reaction system, the N-oxyl compound is gradually oxidized by the co-oxidant to generate an oxammonium salt, by which the cellulose is oxidized. With this oxidation treatment, the oxidation reaction proceeds smoothly even under mild conditions, leading to improvement in the efficiency of introducing a carboxyl group. When the cellulose is subjected to the oxidation treatment under mild conditions, the crystal structure of the cellulose is easily maintained.

The co-oxidant may be any oxidant that can promote the oxidation reaction, such as halogen, hypohalous acid, halous acid, or perhalous acid, or a salt thereof, halogen oxide, nitrogen oxide, or peroxide. In terms of availability and reactivity, sodium hypochlorite is preferable. The amount of co-oxidant to be used is not particularly limited as long as the co-oxidant can promote the oxidation reaction. Typically, the amount of co-oxidant is in the range of approximately 1 to 200 mass% with respect to the solid content of the wood-based natural cellulose subjected to the oxidation treatment.

In combination with the N-oxyl compound and the co-oxidant, at least one compound selected from the group consisting of bromide and iodide may be used. The use of such a compound allows the oxidation reaction to proceed smoothly, leading to improvement in the efficiency of introducing a carboxyl group. The compound is preferably sodium bromide or lithium bromide, and sodium bromide is more preferable in terms of cost and stability. The amount of compound to be used is not particularly limited as long as the compound can promote the oxidation reaction. Typically, the amount of compound is in the range of approximately 1 to 50 mass% with respect to the solid content of the wood-based natural cellulose subjected to the oxidation treatment.

The reaction temperature of the oxidation reaction is preferably in the range of 4°C or more and 80°C or less, and is more preferably in the range of 10°C or more and 70°C or less. If the reaction temperature of the oxidation reaction is less than 4°C, due to reduction in reactivity of a reagent, the reaction time tends to be increased. If the reaction temperature of the oxidation reaction is more than 80°C, due to acceleration of side reactions, the molecular weight of the cellulose as a sample is reduced and the fine cellulose fiber structure that is highly crystalline and rigid is collapsed, and thus the use of the cellulose as the O/W emulsion stabilizer tends to be difficult.

The reaction time of the oxidation treatment is not particularly limited, and may be appropriately set in consideration of the reaction temperature, the desired amount of carboxyl groups, and the like, but is typically in the range of approximately 10 minutes to 5 hours.

The value of pH of the reaction system during the oxidation reaction is not particularly limited, but is preferably in the range of 9 to 11. When the reaction system has a pH of 9 or more, the reaction can proceed efficiently. If the reaction system has a pH of more than 11, side reactions proceed, and thus decomposition of the cellulose as the sample may be accelerated. In the oxidation treatment, as the oxidation proceeds, due to generation of a carboxyl group, the pH in the system is reduced. Thus, during the oxidation treatment, the pH of the reaction system is preferably maintained in the range of 9 to 11. The method of maintaining the pH of the reaction system in the range of 9 to 11 may be, for example, a method in which an alkaline aqueous solution is added according to the reduction of the pH.

Examples of the alkaline aqueous solution include organic alkali such as a sodium hydroxide aqueous solution, a lithium hydroxide aqueous solution, a potassium hydroxide aqueous solution, an ammonia aqueous solution, a tetramethylammonium hydroxide aqueous solution, a tetraethylammonium hydroxide aqueous solution, a tetrabutylammonium hydroxide aqueous solution, and a benzyltrimethylammonium hydroxide aqueous solution. A sodium hydroxide aqueous solution is preferable in terms of cost and the like.

The oxidation reaction using an N-oxyl compound can be stopped, for example, by adding alcohol to the reaction system. In this case, the pH of the reaction system is preferably maintained in the above range. In order to quickly complete the reaction, the alcohol to be added is preferably, for example, a low-molecular-weight alcohol such as methanol, ethanol, or propanol. Ethanol is particularly preferable in terms of safety of by-products generated by the reaction.

The reaction liquid obtained after the oxidation reaction may be provided as it is at the fine-grinding step. However, in order to remove a catalyst such as an N-oxyl compound, impurities, and the like, it is preferable to recover oxidized cellulose contained in the reaction liquid and wash the oxidized cellulose with a cleaning liquid. The oxidized cellulose can be recovered, for example, by a known method such as filtration using a glass filter or a nylon mesh with a pore diameter of 20 µm. The cleaning liquid used to wash the oxidized cellulose is preferably pure water.

By applying defibrating treatment to the obtained TEMPO-oxidized cellulose, cellulose single nanofibers (CSNF) having a uniform fiber width of approximately 3 nm are obtained. When CSNF is used as the raw material of the fine cellulose 1 of the composite particles 5, due to the uniform structure of the CSNF, an O/W emulsion also having a uniform particle size is more likely to be obtained.

As described above, the CSNF used in the present embodiment can be obtained by the step of oxidizing a cellulose raw material and the step of finely grinding the cellulose raw material and forming a dispersion liquid of the cellulose. The content of carboxyl group introduced into the CSNF is preferably in the range of 0.1 mmol/g or more and 5.0 mmol/g or less, and is more preferably in the range of 0.5 mmol/g or more and 2.0 mmol/g or less. If the content of carboxyl group is less than 0.1 mmol/g, solvent entry due to the osmotic pressure effect does not occur between the cellulose microfibrils, and thus fine grinding and uniform dispersion of the cellulose tend to be difficult. If the content of carboxyl group is more than 5.0 mmol/g, due to side reactions accompanying chemical treatment, the molecular weight of the cellulose microfibrils is reduced and the cellulose cannot have a fine cellulose fiber structure that is highly crystalline and rigid, and thus the use of the cellulose as the O/W emulsion stabilizer tends to be difficult.

### (Second step)

The second step is a step of coating at least part of a surface of the biodegradable compound droplet 2 with the fine cellulose 1 in the dispersion liquid 4 of the fine cellulose 1 to stabilize the biodegradable compound droplet 2 as an emulsion.

Specifically, the second step is a step of adding a biodegradable compound to the fine cellulose dispersion liquid obtained at the first step, dispersing the biodegradable compound as droplets in the fine cellulose dispersion liquid, and coating at least part of the surface of the biodegradable compound droplets 2 with the fine cellulose 1, thereby producing an O/W emulsion stabilized by the fine cellulose 1.

Examples of the method of producing an O/W emulsion include a method in which a mixture of a biodegradable compound and a polymerizable monomer containing an initiator is added to the fine cellulose dispersion liquid and emulsified, a method in which a solution obtained by dissolving a biodegradable compound in a solvent having low immiscibility with the fine cellulose dispersion liquid is added to the fine cellulose dispersion liquid and emulsified, and a method in which a biodegradable compound that is solid at normal temperature is added to the fine cellulose dispersion liquid and heated to a melting point of the biodegradable compound or more so that the biodegradable compound is melted and emulsified. In any of the methods, the biodegradable compound is preferably immiscible with the fine cellulose dispersion liquid. If the biodegradable compound is immiscible with the fine cellulose dispersion liquid, the formation of biodegradable compound droplets in the fine cellulose dispersion liquid is difficult, and thus obtaining a complex tends to be difficult.

As the biodegradable compound that can be used in the method in which a mixture of a biodegradable compound and a polymerizable monomer containing an initiator is added to the fine cellulose dispersion liquid and emulsified, the biodegradable compound is not limited and any resin that is not dissolved in water may be used. Specific examples of the biodegradable compound include cellulose acetate derivatives such as cellulose acetate, cellulose acetate butyrate, and cellulose acetate propionate; polysaccharides such as chitin and chitosan; polylactic acids such as polylactic acid and copolymers of lactic acid and other hydroxycarboxylic acids; dibasic acid polyesters such as polybutylene succinate, polyethylene succinate, and polybutylene adipate; polycaprolactones such as polycaprolactone and copolymers of caprolactone and hydroxycarboxylic acid; polyhydroxy butyrates such as polyhydroxy butyrate and copolymers of polyhydroxy butyrate and hydroxycarboxylic acid; aliphatic polyesters such as polyhydroxybutyric acid and copolymers of polyhydroxybutyric acid and other hydroxycarboxylic acids; polyamino acids; polyester-polycarbonates; and natural resins such as rosin. These materials may be used alone or in combination of two or more.

The above biodegradable compound is preferably dissolvable in a polymerizable monomer (described later).

Next, the polymerizable monomer that can be used at the second step will be described.

The type of polymerizable monomer that can be used at the second step is not particularly limited as long as the polymerizable monomer is a monomer of a polymer, has a structure having a polymerizable functional group, is liquid at normal temperature, is completely immiscible with water, and can form a polymer (high molecular weight polymer) by a polymerization reaction. The polymerizable monomer has at least one polymerizable functional group. A polymerizable monomer having a single polymerizable functional group is also referred to as a monofunctional monomer. A polymerizable monomer having two or more polymerizable functional groups is also referred to as a polyfunctional monomer. The type of polymerizable monomer is not particularly limited, and may be, for example, a (meth)acrylic monomer, a vinyl monomer, or the like. The polymerizable monomer may be a polymerizable monomer (e.g., ε-caprolactone, or the like) having a cyclic ether structure such as an epoxy group or an oxetane structure.

The term "(meth)acrylate" includes both "acrylate" and "methacrylate".

Examples of a monofunctional (meth)acrylic monomer include 2-hydroxyethyl (meth)acrylate, 2-hydroxypropyl (meth)acrylate, 2-hydroxybutyl (meth)acrylate, n-butyl (meth)acrylate, isobutyl (meth)acrylate, t-butyl (meth)acrylate, glycidyl (meth)acrylate, acryloylmorpholine, N-vinyl pyrrolidone, tetrahydrofurfuryl acrylate, cyclohexyl (meth)acrylate, 2-ethylhexyl (meth)acrylate, isobornyl (meth)acrylate, isodecyl (meth)acrylate, lauryl (meth)acrylate, tridecyl (meth)acrylate, cetyl (meth)acrylate, stearyl (meth)acrylate, benzyl (meth)acrylate, 2-ethoxyethyl (meth)acrylate, 3-methoxybutyl (meth)acrylate, ethyl carbitol (meth)acrylate, phosphoric acid (meth)acrylate, ethylene oxide modified phosphoric acid (meth)acrylate, phenoxy (meth)acrylate, ethylene oxide modified phenoxy (meth)acrylate, propylene oxide modified phenoxy (meth)acrylate, nonylphenol (meth)acrylate, ethylene oxide modified nonylphenol (meth)acrylate, propylene oxide modified nonylphenol (meth)acrylate, methoxydiethylene glycol (meth)acrylate, methoxypolyethylene glycol (meth)acrylate, methoxypropylene glycol (meth)acrylate, 2-(meth)acryloyloxyethyl-2-hydroxypropyl phthalate, 2-hydroxy-3-phenoxypropyl (meth)acrylate, 2-(meth)acryloyloxyethyl hydrogen phthalate, 2-(meth)acryloyloxypropyl hydrogen phthalate, 2-(meth)acryloyloxypropyl hexahydrohydrogen phthalate, 2-(meth)acryloyloxypropyl tetrahydrohydrogen phthalate, dimethylaminoethyl (meth)acrylate, trifluoroethyl (meth)acrylate, tetrafluoropropyl (meth)acrylate, hexafluoropropyl (meth)acrylate, octafluoropropyl (meth)acrylate, octafluoropropyl (meth)acrylate, and adamantane derivative mono(meth)acrylate such as adamantyl acrylate having monovalent mono(meth)acrylate derived from 2-adamantane and adamantanediol.

Examples of a difunctional (meth)acrylic monomer include di(meth)acrylates such as ethylene glycol di(meth)acrylate, diethylene glycol di(meth)acrylate, butanediol di(meth)acrylate, hexanediol di(meth)acrylate, nonanediol di(meth)acrylate, ethoxylated hexanediol di(meth)acrylate, propoxylated hexanediol di(meth)acrylate, diethylene glycol di(meth)acrylate, polyethylene glycol di(meth)acrylate, tripropylene glycol di(meth)acrylate, polypropylene glycol di(meth)acrylate, neopentyl glycol di(meth)acrylate, ethoxylated neopentyl glycol di(meth)acrylate, tripropylene glycol di(meth)acrylate, and hydroxy pivalic acid neopentyl glycol di(meth)acrylate.

Examples of a trifunctional or higher functional (meth)acrylic monomer include tri(meth)acrylates such as trimethylolpropane tri(meth)acrylate, ethoxylated trimethylolpropane tri(meth)acrylate, propoxylated trimethylolpropane tri(meth)acrylate, tris-2-hydroxyethyl isocyanurate tri(meth)acrylate, and glycerol tri(meth)acrylate; trifunctional (meth)acrylate compounds such as pentaerythritol tri(meth)acrylate, dipentaerythritol tri(meth)acrylate, and ditrimethylolpropane tri(meth)acrylate; trifunctional or higher functional polyfunctional (meth)acrylate compounds such as pentaerythritol tetra(meth)acrylate, ditrimethylolpropane tetra(meth)acrylate, dipentaerythritol tetra(meth)acrylate, dipentaerythritol penta(meth)acrylate, ditrimethylolpropane penta(meth)acrylate, dipentaerythritol hexa(meth)acrylate, and ditrimethylolpropane hexa(meth)acrylate; and polyfunctional (meth)acrylate compounds in which part of these (meth)acrylates is substituted with an alkyl group or ε-caprolactone.

As a monofunctional vinyl monomer, for example, a liquid that is immiscible with water at normal temperature is preferable, such as a vinyl ether monomer, a vinyl ester monomer, an aromatic vinyl monomer, and particularly styrene and a styrene monomer.

Examples of (meth)acrylate of the monofunctional vinyl monomer include methyl (meth)acrylate, ethyl (meth)acrylate, butyl (meth)acrylate, t-butyl (meth)acrylate, 2-ethylhexyl (meth)acrylate, lauryl (meth)acrylate, alkyl (meth)acrylate, tridecyl (meth)acrylate, stearyl (meth)acrylate, cyclohexyl (meth)acrylate, benzyl (meth)acrylate, isobornyl (meth)acrylate, glycidyl (meth)acrylate, tetrahydrofurfuryl (meth)acrylate, allyl (meth)acrylate, diethylaminoethyl (meth)acrylate, trifluoroethyl (meth)acrylate, heptafluorodecyl (meth)acrylate, dicyclopentenyl (meth)acrylate, dicyclopentenyloxyethyl (meth)acrylate, and tricyclodecanyl (meth)acrylate.

Examples of a monofunctional aromatic vinyl monomer include styrene, α-methylstyrene, o-methylstyrene, m-methylstyrene, p-methylstyrene, ethyl styrene, isopropenyl toluene, isobutyl toluene, tert-butylstyrene, vinylnaphthalene, vinylbiphenyl, and 1,1-diphenylethylene.

As a polyfunctional vinyl monomer, for example, a polyfunctional group having an unsaturated bond, such as divinylbenzene may be used. A liquid that is immiscible with water at normal temperature is preferable.

Specific examples of the polyfunctional vinyl monomer include (1) divinyls such as divinylbenzene, 1,2,4-trivinylbenzene, and 1,3,5-trivinylbenzene; (2) dimethacrylates such as ethylene glycol dimethacrylate, diethylene glycol dimethacrylate, triethylene glycol dimethacrylate, polyethylene glycol dimethacrylate, 1,3-propylene glycol dimethacrylate, 1,4-butylene glycol dimethacrylate, 1,6-hexamethylene glycol dimethacrylate, neopentyl glycol dimethacrylate, dipropylene glycol dimethacrylate, polypropylene glycol dimethacrylate, and 2,2-bis(4-methacryloxydiethoxyphenyl)propane; (3) trimethacrylates such as trimethylolpropane trimethacrylate and triethylolethane trimethacrylate; (4) diacrylates such as ethylene glycol diacrylate, diethylene glycol diacrylate, triethylene glycol diacrylate, polyethylene glycol diacrylate, 1,3-dipropylene glycol diacrylate, 1,4-dibutylene glycol diacrylate, 1,6-hexylene glycol diacrylate, neopentyl glycol diacrylate, dipropylene glycol diacrylate, polypropylene glycol diacrylate, 2,2-bis(4-acryloxypropoxyphenyl)propane, and 2,2-bis(4-acryloxydiethoxyphenyl)propane; (5) triacrylates such as trimethylolpropane triacrylate and triethylolethane triacrylate; (6) tetraacrylates such as tetramethylolmethane tetraacrylate; and (7) in addition, for example, tetramethylenebis(ethyl fumarate), hexamethylenebis(acrylamide), triallyl cyanurate, and triallyl isocyanurate.

Specific examples of a functional styrene monomer include divinylbenzene, trivinylbenzene, divinyltoluene, divinylnaphthalene, divinylxylene, divinylbiphenyl, bis(vinylphenyl)methane, bis(vinylphenyl)ethane, bis(vinylphenyl)propane, and bis(vinylphenyl)butane.

Other than these, the polymerizable monomer may be a polyether resin, a polyester resin, a polyurethane resin, an epoxy resin, an alkyd resin, a spiroacetal resin, a polybutadiene resin, a polythiolpolyene resin, or the like having at least one or more polymerizable functional groups, and the material is not particularly limited.

The polymerizable monomers may be used alone or in combination of two or more.

The mixing ratio between the biodegradable compound and the polymerizable monomer is in the range of 10/90 to 85/15, and is preferably in the range of 15/85 to 70/30 in the weight ratio of biodegradable compound/polymerizable monomer. If the proportion of biodegradable compound is less than 10, the mixture tends to have low biodegradability, and if the proportion of polymerizable monomer is less than 15, the formation of a polymer tends to be difficult.

The polymerizable monomer may contain a polymerization initiator added previously. Typical examples of the polymerization initiator include radical initiators such as an organic peroxide and an azo polymerization initiator.

Examples of the organic peroxide include peroxy ketal, hydroperoxide, dialkyl peroxide, diacyl peroxide, peroxy carbonate, and peroxy ester.

The azo polymerization initiator may be, for example, ADVN or AIBN.

Examples of the azo polymerization initiator include 2,2-azobis(isobutyronitrile) (AIBN), 2,2-azobis(2-methylbutyronitrile) (AMBN), 2,2-azobis(2,4-dimethylvaleronitrile) (ADVN), 1,1-azobis(1-cyclohexanecarbonitrile) (ACHN), dimethyl-2,2-azobisisobutylate (MAIB), 4,4-azobis(4-cyanovaleric acid) (ACVA), 1,1-azobis(1-acetoxy-1-phenylethane), 2,2-azobis(2-methylbutylamide), 2,2-azobis(4-methoxy-2,4-dimethylvaleronitrile), 2,2-azobis(2-methylamidinopropane)dihydrochloride, 2,2-azobis[2-(2-imidazoline-2-yl)propane], 2,2-azobis[2-methyl-N-(2-hydroxyethyl)propionamide], 2,2-azobis(2,4,4-trimethylpentane), 2-cyano-2-propylazoformamide, 2,2-azobis(N-butyl-2-methylpropionamide), and 2,2-azobis(N-cyclohexyl-2-methylpropionamide).

In the case where the polymerizable monomer containing a previously-added polymerization initiator is used at the second step, when an O/W emulsion is formed, the polymerization initiator is contained in the polymerizable monomer droplets (biodegradable compound droplets) 2 in the emulsion particles. Thus, when the monomer in the emulsion is polymerized at the third step (described later), the polymerization reaction is more likely to proceed.

The weight ratio between the polymerizable monomer and the polymerization initiator that can be used at the second step is not particularly limited, but typically the weight ratio of the polymerization initiator is preferably 0.1 parts by mass or more with respect to 100 parts by mass of polymerizable monomer. If the weight ratio of the polymerizable monomer is less than 0.1 parts by mass, the polymerization reaction does not sufficiently proceed and the yield of composite particles 5 tends to be reduced, which is not preferable.

As the biodegradable compound that can be used in the method in which a solution obtained by dissolving a biodegradable compound in a solvent having low immiscibility with the fine cellulose dispersion liquid is added to the fine cellulose dispersion liquid and emulsified, the biodegradable compounds described above may be used. The solvent in which the biodegradable compound is dissolved is preferably a solvent having low immiscibility with the fine cellulose dispersion liquid. If the solvent has high solubility in water, the solvent is easily dissolved and transferred from a biodegradable compound phase to an aqueous phase, and thus the emulsification tends to be difficult. If the solvent has no solubility in water, the solvent cannot be transferred from the compound phase, and thus obtaining composite particles tends to be extremely difficult. The solvent in which the biodegradable compound is dissolved preferably has a boiling point of 90°C or less. If the solvent in which the biodegradable compound is dissolved has a boiling point of more than 90°C, the fine cellulose dispersion liquid is evaporated before the solvent, and thus obtaining composite particles tends to be extremely difficult. Specific examples of the solvent in which the biodegradable compound is dissolved include dichloromethane, chloroform, 1,2-dichloroethane, and benzene.

As the biodegradable compound that can be used in the method in which a biodegradable compound that is solid at normal temperature is added to the fine cellulose dispersion liquid and heated to a melting point of the biodegradable compound or more so that the biodegradable compound is melted and emulsified, the biodegradable compound is preferably a biodegradable compound that is solid at normal temperature, is dissolved by heating, and has a melting point in the range of 40°C or more and 90°C or less. If the biodegradable compound has a melting point of less than 40°C, the biodegradable compound is difficult to solidify at normal temperature, and thus obtaining a complex tends to be extremely difficult. If the biodegradable compound has a melting point of 90°C or more, before the biodegradable compound is dissolved, water serving as the solvent of the fine cellulose dispersion liquid heated together with the biodegradable compound is evaporated, and thus the emulsification tends to be difficult. Thus, the biodegradable compound that can be used in the present embodiment may be specifically a biodegradable compound whose main component is pentaerythritol tetrastearate, pentaerythritol distearate, pentaerythritol tristearate, stearyl stearate, batyl stearate, stearyl stearate, myristyl myristate, cetyl palmitate, ethylene glycol distearate, behenyl alcohol, microcrystalline wax, paraffin wax, hydrocarbon wax, fatty acid alkyl ester, polyol fatty acid ester, a mixture of fatty acid ester and wax, a mixture of fatty acid esters, glycerol monopalmitate(/stearic acid monoglyceride), glycerol monodistearate(/glycerol stearate), glycerol monoacetate monostearate(/glycerol fatty acid ester), succinic acid aliphatic monoglyceride(/glycerol fatty acid ester), citric acid saturated aliphatic monoglyceride, sorbitan monostearate, sorbitan fatty acid ester, sorbitan tribehenate, propylene glycol monobehenate(/propylene glycol fatty acid ester), stearate of adipic acid pentaerythritol polymer, pentaerythritol tetrastearate, dipentaerythritol hexastearate, stearyl citrate, pentaerythritol fatty acid ester, glycerol fatty acid ester, ultra-light-colored rosin, rosin-containing diol, ultra-light-colored rosin metal salt, hydrogenated petroleum resin, rosin ester, hydrogenated rosin ester, special rosin ester, novolac, crystalline poly-α-olefin, polyalkylene glycol, polyalkylene glycol ester, or polyoxyalkylene ether.

The biodegradable compound may contain a functional component. Specific examples of the functional component include a magnetic material, a pharmaceutical product, an agricultural chemical, a fragrance, an adhesive, an enzyme, a pigment, a dye, a deodorant, a metal, a metal oxide, and an inorganic oxide. When the biodegradable compound contains a previously-added functional component, the functional component can be contained in particles formed as the composite particles 5, and a function according to the intended use can be exhibited.

The weight ratio between the fine cellulose dispersion liquid and the biodegradable compound and the mixture solution of the biodegradable compound and the polymerizable monomer that can be used at the second step are not particularly limited, but the weight ratio of the biodegradable compound and the mixture solution of the biodegradable compound and the polymerizable monomer is preferably in the range of 1 part by mass or more and 50 parts by mass or less with respect to 100 parts by mass of fine cellulose fibers. If the weight ratio of the biodegradable compound and the mixture solution of the biodegradable compound and the additive is less than 1 part by mass, the yield of composite particles 5 tends to be reduced, which is not preferable. If the weight ratio of the biodegradable compound and the mixture solution of the biodegradable compound and the polymerizable monomer is more than 50 parts by mass, it tends to be difficult to uniformly coat the biodegradable compound droplets 2 with the fine cellulose 1, which is not preferable.

The method of producing an O/W emulsion is not particularly limited, and may be typical emulsification treatment, for example, various types of homogenizer treatment or mechanical stirring treatment. Specifically, the method of producing an O/W emulsion may be mechanical treatment using a high-pressure homogenizer, an ultrahigh-pressure homogenizer, a universal homogenizer, a ball mill, a roll mill, a cutter mill, a planetary mill, a jet mill, an attritor, a grinder, a blender, a homomixer, an ultrasonic homogenizer, a nanogenizer, aqueous counter collision, or a paint shaker. Two or more types of mechanical treatment may be used in combination.

For example, in the case where an ultrasonic homogenizer is used, a polymerizable monomer is added to the fine cellulose dispersion liquid obtained at the first step to form a mixed solvent, and a tip of the ultrasonic homogenizer is inserted into the mixed solvent to perform ultrasonic treatment. The conditions for the ultrasonic homogenizer treatment are not particularly limited, but for example, the frequency is typically 20 kHz or more, and the output is typically 10 W/cm² or more. The treatment time is not particularly limited, but is typically in the range of approximately 10 seconds to 1 hour.

By the ultrasonic treatment, the biodegradable compound droplets 2 are dispersed in the fine cellulose dispersion liquid and emulsified, and the fine cellulose 1 is selectively adsorbed on a liquid-liquid interface between the biodegradable compound droplets 2 and the fine cellulose dispersion liquid. Thus, the biodegradable compound droplets 2 are coated with the fine cellulose 1 to form a stable structure as an O/W emulsion. Such an emulsion that is stabilized by the adsorption of a solid material on a liquid-liquid interface is academically called a "Pickering emulsion". As described above, the mechanism of how fine cellulose fibers form a Pickering emulsion is uncertain, but presumably, since cellulose has a molecular structure including a hydrophilic site derived from the hydroxyl group and a hydrophobic site derived from the hydrocarbon group and thus exhibits amphipathic properties, due to the amphipathic properties, the cellulose is adsorbed on a liquid-liquid interface between the hydrophobic monomer and the hydrophilic solvent.

The O/W emulsion structure can be confirmed, for example, by optical microscope observation. The particle size of the O/W emulsion is not particularly limited, but is typically in the range of approximately 0.1 µm to 1000 µm.

In the O/W emulsion structure, the thickness of a fine cellulose layer (coating layer) formed on a surface layer of the biodegradable compound droplets 2 is not particularly limited, but is typically in the range of approximately 3 nm to 1000 nm. The thickness of the fine cellulose layer can be measured, for example, by using a cryo-TEM.

### (Third step)

The third step is a step of solidifying the biodegradable compound droplet 2 to obtain the composite particle 5 in which the biodegradable compound particle 3 is coated with the fine cellulose 1. More specifically, the third step is a step of, while at least part of the surface of the compound droplet 2 containing the biodegradable compound is coated with the fine cellulose 1, solidifying the droplet 2 containing the biodegradable compound to form the biodegradable compound particle 3 so that at least part of a surface of the biodegradable compound particle 3 is coated with the fine cellulose 1 and the biodegradable compound particle 3 and the fine cellulose 1 are inseparable.

In the case where the second step is performed by the method in which a mixture of a biodegradable compound and a polymerizable monomer containing an initiator is added to the fine cellulose dispersion liquid and emulsified, the method of polymerizing the biodegradable compound and the polymerizable monomer is not particularly limited, and may be appropriately selected according to the types of polymerizable monomer and polymerization initiator used. The method of polymerizing the biodegradable compound and the polymerizable monomer may be, for example, suspension polymerization.

The specific method of the suspension polymerization is not particularly limited, and a known method may be used. For example, the suspension polymerization may be performed by heating while stirring the stabilized O/W emulsion produced at the second step in which the compound droplets 2 containing the biodegradable compound and the polymerizable monomer containing the polymerization initiator are coated with the fine cellulose 1. The stirring method is not particularly limited, and a known method may be used. Specifically, a dispersion device or a stirring bar may be used. Alternatively, the O/W emulsion may be only heated without being stirred. The temperature conditions for heating the O/W emulsion may be appropriately set according to the types of polymerizable monomer and polymerization initiator, but the temperature is preferably in the range of 20°C or more and 90°C or less. During heating of the O/W emulsion, if the temperature is less than 20°C, the reaction rate of polymerization tends to be reduced, which is not preferable. If the temperature is more than 90°C, the fine cellulose dispersion liquid tends to be evaporated, which is not preferable. The time for the polymerization reaction may be appropriately set according to the types of polymerizable monomer and polymerization initiator, but is typically in the range of approximately 1 to 24 hours. The polymerization reaction may be performed by ultraviolet irradiation treatment, i.e., using a type of electromagnetic waves. Other than the electromagnetic waves, a particle beam such as an electron beam may be used.

In the case where the second step is performed by the method in which a solution obtained by dissolving a biodegradable compound in a solvent having low immiscibility with the fine cellulose dispersion liquid is added to the fine cellulose dispersion liquid and emulsified, the biodegradable compound can be solidified by heating the emulsion to volatilize the solvent in which the biodegradable compound is dissolved. The temperature conditions for heating the O/W emulsion may be appropriately set according to the type of solvent in which the biodegradable compound is dissolved, but the temperature is preferably in the range of the boiling point of the solvent or more and 90°C or less. During heating of the O/W emulsion, if the temperature is less than the boiling point of the solvent, the transfer of the solvent to the aqueous phase is delayed, which is not preferable. If the temperature is more than 90°C, the fine cellulose dispersion liquid tends to be evaporated, which is not preferable.

In the case where the second step is performed by the method in which a biodegradable compound that is solid at normal temperature is added to the fine cellulose dispersion liquid and heated to a melting point of the biodegradable compound or more so that the biodegradable compound is melted and emulsified, the biodegradable compound can be solidified by cooling the emulsion to the melting point of the biodegradable compound or less.

Through the steps described above, the spherical composite particles 5 are produced in which the biodegradable compound particles 3 are coated with the fine cellulose 1.

Immediately after the solidification reaction is completed, the dispersion liquid of the composite particles 5 contains both a large amount of water and the fine cellulose 1 that is released and does not contribute to the formation of the coating layer of the composite particles 5. Thus, the produced composite particles 5 need to be recovered and purified. The recovery and purification are preferably performed by centrifugal separation washing or filtration washing. The centrifugal separation washing may be performed by a known method. Specifically, an operation is repeated in which the composite particles 5 are precipitated by centrifugal separation and the supernatant liquid is removed, and the composite particles 5 are redispersed in a mixed solvent of water and methanol. Then, from a precipitate finally obtained by the centrifugal separation, the residual solvent is removed to recover the composite particles 5. The filtration washing may also be performed by a known method. For example, suction filtration with water and methanol is repeatedly performed by using a PTFE membrane filter with a pore diameter of 0.1 µm. Then, from a paste that finally remains on the membrane filter, the residual solvent is removed to recover the composite particles 5.

The method of removing the residual solvent is not particularly limited, and may be performed, for example, by air drying or heat drying in an oven. The dry solid containing the composite particles 5 obtained in this manner is not formed into a film or an aggregate and is obtained as a fine-grained powder as described above.

### (Advantageous effects of embodiment)

The composite particles 5 according to the present embodiment are novel composite particles, having high biocompatibility and good dispersion stability which are derived from the fine cellulose 1 on the surface thereof, with the good dispersion stability allowing the composite particles 5 to be prevented from aggregating in a solvent.

The dry solid containing the composite particles 5 according to the present embodiment is obtained as a fine-grained powder with no aggregation of the particles. Thus, the composite particles 5 obtained as a dry powder are easily redispersed in a solvent, and after the redispersion, the dispersion liquid also exhibits dispersion stability derived from the coating layer of the fine cellulose 1 bonded to the surface of the composite particles 5.

According to the method of producing the composite particles 5 of the present embodiment, it is possible to provide a method of producing the novel composite particles 5 that can be provided by a simple method with low environmental load.

According to the dry solid containing the complex of the fine cellulose 1 of the present embodiment, it is possible to provide a dry solid redispersible in a solvent.

The composite particles 5 according to the present embodiment allow most of the solvent to be removed, and are thus expected to achieve effects such as reduction in transport cost, reduction in risk of spoilage, improvement in addition efficiency as an additive, and improvement in efficiency of kneading with hydrophobic resin.

The embodiment of the present invention has been described in detail above with reference to the drawings. However, the specific configuration should not be limited to the embodiment, but should include design changes within the scope not departing from the spirit of the present invention. Furthermore, the components described in the first embodiment and the modifications described above can be combined as appropriate.

### [Second embodiment]

### <Composite particle of fine cellulose and polymer>

First, a composite particle 5 of fine cellulose and a polymer particle contained in a skin application composition according to a second embodiment of the present invention will be described. Fig. 1 is a schematic diagram of an O/W Pickering emulsion containing a cellulose nanofiber (hereinafter also referred to as CNF or cellulose) 1, and the composite particle 5 obtained by solidifying a polymerizable monomer droplet and/or polymer droplet 2 (hereinafter also simply referred to as "droplet 2") in the emulsion.

The composite particle 5 is a composite particle that contains at least one type of polymer particle 3 and has a coating layer composed of the fine cellulose 1 and provided on a surface of the polymer particle 3 and in which the polymer particle 3 and the fine cellulose 1 are bonded to each other and are inseparable.

As shown in Fig. 1, the cellulose 1 is adsorbed on an interface of the polymerizable monomer droplets and/or polymer droplets 2 dispersed in a dispersion liquid 4 to stabilize the O/W Pickering emulsion. While the stabilized state of the O/W Pickering emulsion is maintained, the droplets 2 in the emulsion are solidified to produce the composite particles 5 by using the emulsion as a template.

The solidification of the polymerizable monomer droplets and/or polymer droplets 2 includes (1) polymerization of the polymerizable monomer droplets, (2) solidification of the polymer droplets, and (3) solidification of the polymerizable monomer droplets and the polymer droplets.

The term "inseparable" means a state in which even after repetition of an operation of centrifuging a dispersion liquid containing the composite particles 5 and removing the supernatant liquid, followed by addition of a solvent and redispersion to purify and wash the composite particles 5 or an operation of repeatedly washing the composite particles 5 with a solvent by filtration washing using a membrane filter, the fine cellulose 1 and the polymer particles 3 are not separated and the coating state is maintained in which the polymer particles 3 are coated with the fine cellulose 1. The coating state can be confirmed by observing a surface of the composite particle 5 by using a scanning electron microscope. The mechanism of how the fine cellulose 1 is bonded to the polymer particle 3 in the composite particle 5 is uncertain, but presumably, since the composite particle 5 is produced by using, as a template, the O/W emulsion stabilized by the fine cellulose 1, the droplet 2 in the emulsion is solidified while the fine cellulose 1 is in contact with the droplet 2, and thus the fine cellulose 1 is physically immobilized on the droplet 2 that is being solidified, and the polymer particle 3 and the fine cellulose 1 become inseparable as a result.

The O/W emulsion is also referred to as oil-in-water emulsion, and is an emulsion containing water as a continuous phase in which oil in the form of oil droplets (oil particles) is dispersed.

Since the composite particle 5 is produced by using, as a template, the O/W emulsion stabilized by the fine cellulose 1, the composite particle 5 is characterized by having a spherical shape derived from the O/W emulsion. Specifically, a coating layer composed of the fine cellulose 1 is formed with a relatively uniform thickness on the surface of the spherical polymer particles 3. The average thickness of the coating layer can be calculated in the following manner. That is, the composite particle 5 fixed with an embedding resin is cut with a microtome and observed by using a scanning electron microscope. In an image, the thickness of the coating layer in a cross-sectional image of the composite particle 5 is randomly measured at 100 locations, and the average value of the thicknesses is obtained. The composite particle 5 is characterized by being uniformly coated with the coating layer having a relatively uniform thickness. Specifically, the coefficient of variation of the thickness of the coating layer is preferably 0.5 or less, and is more preferably 0.4 or less.

The fine cellulose 1 of the present embodiment is not particularly limited, but preferably has an anionic functional group on the crystal surface. The content of anionic functional group is preferably in the range of 0.1 mmol or more and 5.0 mmol or less per 1 g of cellulose.

Furthermore, the fine cellulose 1 preferably has a fiber form derived from a microfibril structure. Specifically, the fine cellulose 1 is preferably fibrous, and has a number average minor axis diameter in the range of 1 nm or more and 1000 nm or less, and a number average major axis diameter of 50 nm or more. The number average major axis diameter is preferably 5 times or more the number average minor axis diameter. In addition, the fine cellulose 1 preferably has a crystal structure of cellulose type I.

### <Method of producing composite particle>

Next, a method of producing a composite particle contained in a skin application composition of the present embodiment will be described. The method of producing a composite particle according to the present embodiment is a method of producing the composite particle 5 including: a step (first step) of defibrating a cellulose raw material in a solvent to obtain a dispersion liquid of a fine cellulose; a step (second step) of coating a surface of the polymerizable monomer droplet and/or polymer droplet 2 with the fine cellulose 1 in the dispersion liquid of the fine cellulose to stabilize the polymerizable monomer droplet and/or polymer droplet 2 as an emulsion; and a step (third step) of solidifying the droplet 2 to obtain the composite particle 5 in which the polymer particle 3 is coated with the fine cellulose 1.

The composite particles 5 obtained by the above production method are obtained as a dispersion. By removing the solvent, the composite particles 5 are obtained as a dry solid. The method of removing the solvent is not particularly limited, and the composite particles 5 can be obtained as a dry solid, for example, by removing excess moisture by centrifugal separation or filtration, followed by heat drying in an oven. In this case, the dry solid is not formed into a film or an aggregate and is obtained as a fine-grained powder. The reason is uncertain, but it is known that when a solvent is removed from a fine cellulose dispersion, typically, pieces of fine cellulose are strongly aggregated and formed into a film. On the other hand, in the case of the dispersion liquid containing the composite particles 5, since the composite particles 5 are spherical composite particles having a surface on which the fine cellulose 1 is immobilized, even when the solvent is removed, pieces of fine cellulose 1 are not aggregated and the composite particles are only in point contact with each other. This is presumably the reason why the dry solid is obtained as a fine-grained powder. Thus, the composite particles of the present invention, which can be used as a fine-grained powder, are suitable, for example, as a composition for a powder foundation. Furthermore, since the composite particles 5 are not aggregated, the composite particles 5 obtained as a dry powder are easily redispersed in a solvent, and after the redispersion, the dispersion liquid also exhibits dispersion stability derived from the fine cellulose 1 bonded to the surface of the composite particles 5. In this case, an anionic functional group is preferably introduced to the crystal surface of the fine cellulose 1 to be used. This is because, in that case, the anionic functional group is selectively arranged on the surface of the composite particles and the solvent is more likely to enter between the composite particles due to the osmotic pressure effect, leading to further improvement in the dispersion stability.

The dry powder of the composite particles 5 is a dry solid having an advantage of containing little solvent and being redispersible in a solvent. Specifically, the dry powder may have a solid content ratio of 80% or more, 90% or more, or 95% or more. Since most of the solvent can be removed, the dry powder of the composite particles 5 has advantageous effects in terms of reduction in transport cost, prevention of spoilage, and improvement in addition ratio. When the dry powder having a solid content ratio of 80% or more is obtained by drying treatment, the fine cellulose 1 easily absorbs moisture, and thus may adsorb moisture in air and cause reduction of the solid content ratio over time. However, considering the technical idea of the present invention having an advantage that the composite particles 5 are easily obtained as a dry powder and the dry powder is redispersible, a dry solid including a step of causing a dry powder containing the composite particles 5 to have a solid content ratio of 80% or more is defined to be encompassed in the technical scope of the present invention.

The steps will be described below in detail.

### (First step)

The first step is a step of defibrating a cellulose raw material in a solvent to obtain a fine cellulose dispersion liquid. First, a cellulose raw material is dispersed in a solvent to form a suspension. The cellulose raw material is preferably contained in the suspension at a concentration in the range of 0.1% or more and less than 10%. If the cellulose raw material is contained in the suspension at a concentration of less than 0.1%, due to the excessive solvent, productivity is deteriorated, which is not preferable. If the cellulose raw material is contained in the suspension at a concentration of 10% or more, due to the defibration of the cellulose raw material, the suspension is rapidly thickened, and thus uniform defibrating treatment is difficult, which is not preferable. The solvent used to produce a suspension preferably contains 50% or more of water. If the ratio of water in the suspension is 50% or less, at the step of defibrating a cellulose raw material in a solvent to obtain a fine cellulose dispersion liquid (described later), dispersion of the fine cellulose 1 is inhibited. A solvent other than water contained in the suspension is preferably a hydrophilic solvent. The hydrophilic solvent is not particularly limited, but is preferably an alcohol such as methanol, ethanol, and isopropanol; or cyclic ethers such as tetrahydrofuran. If necessary, the pH of the suspension may be adjusted in order to improve dispersibility of the cellulose and the fine cellulose 1 to be generated. Examples of an alkaline aqueous solution used for the pH adjustment include organic alkali such as a sodium hydroxide aqueous solution, a lithium hydroxide aqueous solution, a potassium hydroxide aqueous solution, an ammonia aqueous solution, a tetramethylammonium hydroxide aqueous solution, a tetraethylammonium hydroxide aqueous solution, a tetrabutylammonium hydroxide aqueous solution, and a benzyltrimethylammonium hydroxide aqueous solution. A sodium hydroxide aqueous solution is preferable in terms of cost and the like.

Next, the suspension is subject to physical defibrating treatment to finely grind the cellulose raw material. The physical defibrating treatment is not particularly limited, and may be mechanical treatment using a high-pressure homogenizer, an ultrahigh-pressure homogenizer, a ball mill, a roll mill, a cutter mill, a planetary mill, a jet mill, an attritor, a grinder, a blender, a homomixer, an ultrasonic homogenizer, a nanogenizer, or aqueous counter collision. By the physical defibrating treatment, the cellulose in the suspension is finely ground to form a dispersion liquid of the cellulose 1 finely ground until at least one dimension of a structure of the cellulose has a nanometer-order size. The treatment time and the number of times the physical defibrating treatment is performed can be adjusted to control the number average minor axis diameter and the number average major axis diameter of the fine cellulose 1 to be obtained.

In this manner, a dispersion (fine cellulose dispersion liquid) of the cellulose 1 finely ground until at least one dimension of the structure of the cellulose has a nanometer-order size is obtained. The obtained dispersion can be used as an O/W emulsion stabilizer (described later) as it is or after dilution, concentration, or the like.

In addition to the cellulose and the component used for the pH adjustment, the fine cellulose dispersion may contain an additional component as necessary to such an extent that the effects of the present invention are not impaired. The additional component is not particularly limited, and may be appropriately selected from known additives according to the intended use of the composite particles 5 or the like. Specific examples of the additional component include an organometallic compound such as alkoxysilane or a hydrolysate thereof, an inorganic layered compound, an inorganic needle-like mineral, an antifoaming agent, inorganic particles, organic particles, a lubricant, an antioxidant, an antistatic agent, an ultraviolet absorber, a stabilizer, a magnetic powder, an orientation accelerator, a plasticizer, a crosslinking agent, a magnetic material, a pharmaceutical product, an agricultural chemical, a fragrance, an adhesive, an enzyme, a pigment, a dye, a deodorant, a metal, a metal oxide, and an inorganic oxide.

Since the fine cellulose 1 typically has a fiber form derived from a microfibril structure, the fine cellulose 1 used in the production method of the present embodiment preferably has a fiber form with a size in the following range. That is, the form of the fine cellulose 1 is preferably fibrous. Furthermore, the fibrous fine cellulose 1 preferably has a number average minor axis diameter in the range of 1 nm or more and 1000 nm or less, and more preferably in the range of 2 nm or more and 500 nm or less. If the fine cellulose 1 has a number average minor axis diameter of less than 1 nm, the fine cellulose 1 cannot have a fine cellulose fiber structure that is highly crystalline and rigid, and thus stabilization of the emulsion and a polymerization reaction using the emulsion as a template cannot be performed. On the other hand, if the fine cellulose 1 has a number average minor axis diameter of more than 1000 nm, the fine cellulose 1 is excessively large in size for stabilization of the emulsion, and thus control of the size and shape of the composite particles 5 to be obtained is difficult. Furthermore, the number average major axis diameter of the fine cellulose 1 is not particularly limited, but is preferably 5 times or more the number average minor axis diameter. If the fine cellulose 1 has a number average major axis diameter of less than 5 times the number average minor axis diameter, the size and shape of the composite particles 5 cannot be sufficiently controlled, which is not preferable.

The number average minor axis diameter of the fine cellulose fibers is obtained by measuring minor axis diameters (minimum diameters) of 100 fibers by transmission electron microscope observation or atomic force microscope observation, and calculating an average value of the minor axis diameters. On the other hand, the number average major axis diameter of the fine cellulose fibers is obtained by measuring major axis diameters (maximum diameters) of 100 fibers by transmission electron microscope observation or atomic force microscope observation, and calculating an average value of the major axis diameters.

The type and crystal structure of cellulose that can be used as the raw material of the fine cellulose 1 are not particularly limited. Specific examples of a raw material composed of cellulose I type crystals include, in addition to wood-based natural cellulose, non-wood-based natural cellulose such as cotton linters, bamboo, hemp, bagasse, kenaf, bacterial cellulose, hoya cellulose, and valonia cellulose. Furthermore, the raw material of the fine cellulose 1 may also be regenerated cellulose typified by rayon fibers and cuprammonium rayon fibers composed of cellulose II type crystals. Wood-based natural cellulose is preferable as the raw material in terms of ease of material acquisition. The wood-based natural cellulose is not particularly limited, and may be wood-based natural cellulose typically used to produce cellulose nanofibers, such as softwood pulp, hardwood pulp, or waste paper pulp. Softwood pulp is preferable in terms of ease of purification and fine grinding.

Furthermore, the fine cellulose raw material is preferably chemically modified. More specifically, an anionic functional group is preferably introduced to a crystal surface of the fine cellulose raw material. This is because when an anionic functional group is introduced to the crystal surface of the cellulose, the solvent is more likely to enter between the cellulose crystals due to the osmotic pressure effect, thereby facilitating fine grinding of the cellulose raw material.

The type of anionic functional group introduced to the crystal surface of the cellulose and the introduction method are not particularly limited, but a carboxyl group or a phosphate group is preferable. A carboxyl group is preferable in terms of ease of selective introduction to the crystal surface of the cellulose.

The method of introducing a carboxyl group to the surface of the cellulose fibers is not particularly limited. Specifically, for example, the cellulose may be allowed to react with monochloroacetic acid or sodium monochloroacetate in a high-concentration alkaline aqueous solution so as to be carboxymethylated. Alternatively, to introduce a carboxyl group, the cellulose may be allowed to directly react with a carboxylic acid anhydride compound such as maleic acid or phthalic acid gasified in an autoclave. Alternatively, a carboxyl group may be introduced by a method in which a co-oxidant is used in the presence of an N-oxyl compound, such as TEMPO, which has high selectivity for oxidation of alcoholic primary carbon, while maintaining the structure as much as possible under relatively mild aqueous conditions. The oxidation using an N-oxyl compound is more preferable in terms of selectivity for a portion to which the carboxyl group is introduced and reduction of the environmental load.

Examples of the N-oxyl compound include TEMPO (2,2,6,6-tetramethylpiperidinyl-1-oxy radical), 2,2,6,6-tetramethyl-4-hydroxypiperidin-1-oxyl, 4-methoxy-2,2,6,6-tetramethylpiperidin-N-oxyl, 4-ethoxy-2,2,6,6-tetramethylpiperidin-N-oxyl, and 4-acetamide-2,2,6,6-tetramethylpiperidin-N-oxyl. Among these, TEMPO which has high reactivity is preferable. The amount of N-oxyl compound to be used is not particularly limited as long as the N-oxyl compound is contained in the amount required for use as a catalyst. Typically, the amount of N-oxyl compound is in the range of approximately 0.01 to 5.0 mass% with respect to the solid content of the wood-based natural cellulose subjected to the oxidation treatment.

The oxidation method using an N-oxyl compound may be, for example, a method in which wood-based natural cellulose is dispersed in water and subjected to oxidation treatment in the presence of the N-oxyl compound. In this method, it is preferable to use a co-oxidant in combination with the N-oxyl compound. In this case, in the reaction system, the N-oxyl compound is gradually oxidized by the co-oxidant to generate an oxammonium salt, by which the cellulose is oxidized. With this oxidation treatment, the oxidation reaction proceeds smoothly even under mild conditions, leading to improvement in the efficiency of introducing a carboxyl group. When the cellulose is subjected to the oxidation treatment under mild conditions, the crystal structure of the cellulose is easily maintained.

The co-oxidant may be any oxidant that can promote the oxidation reaction, such as halogen, hypohalous acid, halous acid, or perhalous acid, or a salt thereof, halogen oxide, nitrogen oxide, or peroxide. In terms of availability and reactivity, sodium hypochlorite is preferable. The amount of co-oxidant to be used is not particularly limited as long as the co-oxidant can promote the oxidation reaction. Typically, the amount of co-oxidant is in the range of approximately 1 to 200 mass% with respect to the solid content of the wood-based natural cellulose subjected to the oxidation treatment.

In combination with the N-oxyl compound and the co-oxidant, at least one compound selected from the group consisting of bromide and iodide may be used. The use of such a compound allows the oxidation reaction to proceed smoothly, leading to improvement in the efficiency of introducing a carboxyl group. The compound is preferably sodium bromide or lithium bromide, and sodium bromide is more preferable in terms of cost and stability. The amount of compound to be used is not particularly limited as long as the compound can promote the oxidation reaction. Typically, the amount of compound is in the range of approximately 1 to 50 mass% with respect to the solid content of the wood-based natural cellulose subjected to the oxidation treatment.

The reaction temperature of the oxidation reaction is preferably in the range of 4°C to 80°C, and is more preferably in the range of 10°C to 70°C. If the reaction temperature of the oxidation reaction is less than 4°C, due to reduction in reactivity of a reagent, the reaction time is increased. If the reaction temperature of the oxidation reaction is more than 80°C, due to acceleration of side reactions, the molecular weight of the sample is reduced and the fine cellulose fiber structure that is highly crystalline and rigid is collapsed, and thus the sample cannot be used as the O/W emulsion stabilizer.

The reaction time of the oxidation treatment is not particularly limited, and may be appropriately set in consideration of the reaction temperature, the desired amount of carboxyl groups, and the like, but is typically in the range of approximately 10 minutes to 5 hours.

The value of pH of the reaction system during the oxidation reaction is not particularly limited, but is preferably in the range of 9 to 11. When the reaction system has a pH of 9 or more, the reaction can proceed efficiently. If the reaction system has a pH of more than 11, side reactions proceeds, and thus decomposition of the sample may be accelerated. In the oxidation treatment, as the oxidation proceeds, due to generation of a carboxyl group, the pH in the system is reduced. Thus, during the oxidation treatment, the pH of the reaction system is preferably maintained in the range of 9 to 11. The method of maintaining the pH of the reaction system in the range of 9 to 11 may be a method in which an alkaline aqueous solution is added according to the reduction of the pH.

Examples of the alkaline aqueous solution include organic alkali such as a sodium hydroxide aqueous solution, a lithium hydroxide aqueous solution, a potassium hydroxide aqueous solution, an ammonia aqueous solution, a tetramethylammonium hydroxide aqueous solution, a tetraethylammonium hydroxide aqueous solution, a tetrabutylammonium hydroxide aqueous solution, and a benzyltrimethylammonium hydroxide aqueous solution. A sodium hydroxide aqueous solution is preferable in terms of cost and the like.

The oxidation reaction using an N-oxyl compound can be stopped by adding alcohol to the reaction system. In this case, the pH of the reaction system is preferably maintained in the above range. In order to quickly complete the reaction, the alcohol to be added is preferably a low-molecular-weight alcohol such as methanol, ethanol, or propanol. Ethanol is particularly preferable in terms of safety of by-products generated by the reaction.

The reaction liquid obtained after the oxidation reaction may be provided as it is at the fine-grinding step. However, in order to remove a catalyst such as an N-oxyl compound, impurities, and the like, it is preferable to recover oxidized cellulose contained in the reaction liquid and wash the oxidized cellulose with a cleaning liquid. The oxidized cellulose can be recovered by a known method such as filtration using a glass filter or a nylon mesh with a pore diameter of 20 µm. The cleaning liquid used to wash the oxidized cellulose is preferably pure water.

By applying defibrating treatment to the obtained TEMPO-oxidized cellulose, cellulose single nanofibers (CSNF) having a uniform fiber width of 3 nm are obtained. When CSNF is used as the raw material of the fine cellulose 1 of the composite particles 5, due to the uniform structure of the CSNF, an O/W emulsion also having a uniform particle size is more likely to be obtained.

As described above, the CSNF used in the present embodiment can be obtained by the step of oxidizing a cellulose raw material and the step of finely grinding the cellulose raw material and forming a dispersion liquid of the cellulose. The content of carboxyl group introduced into the CSNF is preferably in the range of 0.1 mmol/g or more and 5.0 mmol/g or less, and is more preferably in the range of 0.5 mmol/g or more and 2.0 mmol/g or less. If the content of carboxyl group is less than 0.1 mmol/g, solvent entry due to the osmotic pressure effect does not occur between the cellulose microfibrils, and thus fine grinding and uniform dispersion of the cellulose are difficult. If the content of carboxyl group is more than 5.0 mmol/g, due to side reactions accompanying chemical treatment, the molecular weight of the cellulose microfibrils is reduced and the cellulose cannot have a fine cellulose fiber structure that is highly crystalline and rigid, and thus the cellulose cannot be used as the O/W emulsion stabilizer.

### (Second step)

The second step is a step of coating a surface of the polymerizable monomer droplet and/or polymer droplet 2 with the fine cellulose 1 in the dispersion liquid of the fine cellulose to stabilize the polymerizable monomer droplet and/or polymer droplet 2 as an emulsion.

Specifically, the second step is a step of adding a polymerizable monomer and/or molten polymer to the fine cellulose dispersion liquid obtained at the first step, dispersing the polymerizable monomer and/or molten polymer as droplets 2 in the fine cellulose dispersion liquid, and coating the surface of the droplets 2 with the fine cellulose 1, thereby producing an O/W emulsion stabilized by the fine cellulose 1.

The method of producing an O/W emulsion is not particularly limited, and may be typical emulsification treatment, for example, various types of homogenizer treatment or mechanical stirring treatment. Specifically, the method of producing an O/W emulsion may be mechanical treatment using a high-pressure homogenizer, an ultrahigh-pressure homogenizer, a universal homogenizer, a ball mill, a roll mill, a cutter mill, a planetary mill, a jet mill, an attritor, a grinder, a blender, a homomixer, an ultrasonic homogenizer, a nanogenizer, aqueous counter collision, or a paint shaker. Two or more types of mechanical treatment may be used in combination.

For example, in the case where an ultrasonic homogenizer is used, a polymerizable monomer and/or molten polymer is added to the fine cellulose dispersion liquid obtained at the first step to form a mixed solvent, and a tip of the ultrasonic homogenizer is inserted into the mixed solvent to perform ultrasonic treatment. The conditions for the ultrasonic homogenizer treatment are not particularly limited, but for example, the frequency is typically 20 kHz or more, and the output is typically 10 W/cm² or more. The treatment time is not particularly limited, but is typically in the range of approximately 10 seconds to 1 hour.

By the ultrasonic treatment, the polymerizable monomer droplets and/or polymer droplets 2 are dispersed in the fine cellulose dispersion liquid and emulsified, and the fine cellulose 1 is selectively adsorbed on a liquid-liquid interface between the droplets 2 and the fine cellulose dispersion liquid. Thus, the droplets 2 are coated with the fine cellulose 1 to form a stable structure as an O/W emulsion. Such an emulsion that is stabilized by the adsorption of a solid material on a liquid-liquid interface is academically called a "Pickering emulsion". As described above, the mechanism of how fine cellulose fibers form a Pickering emulsion is uncertain, but presumably, since cellulose has a molecular structure including a hydrophilic site derived from the hydroxyl group and a hydrophobic site derived from the hydrocarbon group and thus exhibits amphipathic properties, due to the amphipathic properties, the cellulose is adsorbed on a liquid-liquid interface between the hydrophobic monomer and the hydrophilic solvent.

The O/W emulsion structure can be confirmed by optical microscope observation. The particle size of the O/W emulsion is not particularly limited, but is typically in the range of approximately 0.1 µm to 1000 µm.

In the O/W emulsion structure, the thickness of a fine cellulose layer formed on a surface layer of the droplets 2 is not particularly limited, but is typically in the range of approximately 3 nm to 1000 nm. The thickness of the fine cellulose layer can be measured, for example, by using a cryo-TEM.

The type of polymerizable monomer that can be used at the second step is not particularly limited as long as the polymerizable monomer is a monomer of a polymer, has a structure having a polymerizable functional group, is liquid at normal temperature, is immiscible with water, and can form a polymer (high molecular weight polymer) by a polymerization reaction. The polymerizable monomer has at least one polymerizable functional group. A polymerizable monomer having a single polymerizable functional group is also referred to as a monofunctional monomer. A polymerizable monomer having two or more polymerizable functional groups is also referred to as a polyfunctional monomer. The type of polymerizable monomer is not particularly limited, and may be, for example, a (meth)acrylic monomer, a vinyl monomer, or the like. The polymerizable monomer may be a polymerizable monomer (e.g., ε-caprolactone, or the like) having a cyclic ether structure such as an epoxy group or an oxetane structure.

The term "(meth)acrylate" includes both "acrylate" and "methacrylate".

Examples of a monofunctional (meth)acrylic monomer, a difunctional (meth)acrylic monomer, a trifunctional or higher functional (meth)acrylic monomer, a monofunctional vinyl monomer, a monofunctional aromatic vinyl monomer, a polyfunctional vinyl monomer, and a functional styrene monomer include those listed in the first embodiment.

Other than these, the polymerizable monomer may be a polyether resin, a polyester resin, a polyurethane resin, an epoxy resin, an alkyd resin, a spiroacetal resin, a polybutadiene resin, a polythiolpolyene resin, or the like having at least one or more polymerizable functional groups, and the material is not particularly limited.

The polymerizable monomers may be used alone or in combination of two or more.

The weight ratio between the fine cellulose fiber dispersion liquid and the polymerizable monomer that can be used at the second step is not particularly limited, but the weight ratio of the polymerizable monomer is preferably in the range of 1 part by mass or more and 50 parts by mass or less with respect to 100 parts by mass of fine cellulose fibers. If the weight ratio of the polymerizable monomer is 1 part by mass or less per 100 parts by mass of fine cellulose fiber, the yield of composite particles 5 is reduced, which is not preferable. If the polymerizable monomer is more than 50 parts by mass, it is difficult to uniformly coat the polymerizable monomer droplets with the fine cellulose 1, which is not preferable.

The polymerizable monomer may contain a polymerization initiator added previously. Typical examples of the polymerization initiator include radical initiators such as an organic peroxide and an azo polymerization initiator.

Examples of the organic peroxide and the azo polymerization initiator include those listed in the first embodiment.

In the case where the polymerizable monomer containing a previously-added polymerization initiator is used at the second step, when an O/W emulsion is formed, the polymerization initiator is contained in the polymerizable monomer droplets in the emulsion particles. Thus, when the monomer in the emulsion is polymerized and solidified at the third step (described later), the polymerization reaction is more likely to proceed.

The weight ratio between the polymerizable monomer and the polymerization initiator that can be used at the second step is not particularly limited, but typically the weight ratio of the polymerization initiator is preferably 0.1 parts by mass or more with respect to 100 parts by mass of polymerizable monomer. If the weight ratio of the polymerizable monomer is less than 0.1 parts by mass, the polymerization reaction does not sufficiently proceed and the yield of composite particles 5 is reduced, which is not preferable.

The droplets 2 that can be used at the second step may be dissolved polymer droplets obtained by dissolving an existing polymer by using various solvents. For example, the droplets 2 are preferably prepared in the following manner. That is, an existing polymer is dissolved in a solvent having low immiscibility with the fine cellulose dispersion liquid to form a solution. Then, as described above, while the solution is subjected to mechanical treatment using an ultrasonic homogenizer or the like, the solution is added to a CNF dispersion liquid to stabilize the polymer droplets as an O/W emulsion in the CNF dispersion liquid.

Specific examples of the polymer include cellulose acetate derivatives such as cellulose acetate, cellulose acetate butyrate, and cellulose acetate propionate; polysaccharides such as chitin and chitosan; polylactic acids such as polylactic acid and copolymers of lactic acid and other hydroxycarboxylic acids; dibasic acid polyesters such as polybutylene succinate, polyethylene succinate, and polybutylene adipate; polycaprolactones such as polycaprolactone and copolymers of caprolactone and hydroxycarboxylic acid; polyhydroxy butyrates such as polyhydroxy butyrate and copolymers of polyhydroxy butyrate and hydroxycarboxylic acid; aliphatic polyesters such as polyhydroxybutyric acid and copolymers of polyhydroxybutyric acid and other hydroxycarboxylic acids; polyamino acids; polyester-polycarbonates; and natural resins such as rosin. These materials may be used alone or in combination of two or more.

A polymer that is particularly suitable for a skin application composition is, for example, polylactic acid which is derived from plants, is biodegradable, and is also used as a medical material.

The solvent in which the polymer is dissolved is preferably a solvent having low immiscibility with the fine cellulose dispersion liquid. If the solvent has high solubility in water, the solvent is easily dissolved and transferred from a dissolved polymer droplet phase to an aqueous phase, and thus the emulsification is difficult. On the other hand, if the solvent has no solubility in water, the solvent cannot be transferred from the dissolved polymer droplet phase to a fine cellulose dispersion liquid phase, and thus no composite particles can be obtained. The solvent preferably has a boiling point of 90°C or less. If the solvent has a boiling point of more than 90°C, the fine cellulose dispersion liquid is evaporated before the solvent, and thus no composite particles can be obtained. Specific examples of the solvent that can be used include dichloromethane, chloroform, 1,2-dichloroethane, and benzene.

At the second step, instead of using the solvent, molten polymer droplets obtained by melting a polymer may be used. For example, the molten polymer droplets are preferably prepared in the following manner. That is, a polymer that is solid at normal temperature is melted to form a liquid. Then, as described above, while the molten liquid is subjected to mechanical treatment using an ultrasonic homogenizer or the like, the molten liquid is added to a CNF dispersion liquid heated to a temperature at which the molten state of the polymer can be maintained, thereby stabilizing the polymer droplets as an O/W emulsion in the CNF dispersion liquid.

The molten polymer droplets that can be used at the second step preferably have low solubility in the fine cellulose water dispersion liquid. If the molten polymer droplets have high solubility in water, the polymer is easily dissolved and transferred from a molten polymer droplet phase to an aqueous phase, and thus emulsification is difficult. The molten polymer preferably has a melting point of 90°C or less. If the molten polymer has a melting point of more than 90°C, water in the fine cellulose dispersion liquid is evaporated, and thus the emulsification is difficult. Specific examples of the molten polymer include pentaerythritol tetrastearate, pentaerythritol distearate, pentaerythritol tristearate, stearyl stearate, batyl stearate, stearyl stearate, myristyl myristate, cetyl palmitate, ethylene glycol distearate, behenyl alcohol, microcrystalline wax, paraffin wax, hydrocarbon wax, fatty acid alkyl ester, polyol fatty acid ester, a mixture of fatty acid ester and wax, a mixture of fatty acid esters, glycerol monopalmitate(/stearic acid monoglyceride), glycerol monodistearate(/glycerol stearate), glycerol monoacetate monostearate(/glycerol fatty acid ester), succinic acid aliphatic monoglyceride(/glycerol fatty acid ester), citric acid saturated aliphatic monoglyceride, sorbitan monostearate, sorbitan fatty acid ester, sorbitan tribehenate, propylene glycol monobehenate(/propylene glycol fatty acid ester), stearate of adipic acid pentaerythritol polymer, pentaerythritol tetrastearate, dipentaerythritol hexastearate, stearyl citrate, pentaerythritol fatty acid ester, glycerol fatty acid ester, ultra-light-colored rosin, rosin-containing diol, ultra-light-colored rosin metal salt, hydrogenated petroleum resin, rosin ester, hydrogenated rosin ester, special rosin ester, novolac, crystalline poly-α-olefin, polyalkylene glycol, polyalkylene glycol ester, polyoxyalkylene ether, polylactic acids such as polylactic acid and copolymers of lactic acid and other hydroxycarboxylic acids, dibasic acid polyesters such as polybutylene succinate, polyethylene succinate, and polybutylene adipate, polycaprolactones such as polycaprolactone and copolymers of caprolactone and hydroxycarboxylic acid, polyhydroxy butyrates such as polyhydroxy butyrate and copolymers of polyhydroxy butyrate and hydroxycarboxylic acid, aliphatic polyesters such as polyhydroxybutyric acid and copolymers of polyhydroxybutyric acid and other hydroxycarboxylic acids, polyamino acids, polyester-polycarbonates, and natural resins such as rosin.

The polymerizable monomer droplets and/or polymer droplets 2 may contain a previously-added functional component other than the polymerization initiator. Specific examples of the functional component include a magnetic material, a pharmaceutical product, an agricultural chemical, a fragrance, an adhesive, an enzyme, a pigment, a dye, a deodorant, a metal, a metal oxide, and an inorganic oxide. When the polymerizable monomer contains a previously-added functional component other than the polymerization initiator, the functional component can be contained in particles formed as the composite particles 5, and a function according to the intended use can be exhibited.

Furthermore, the polymerizable monomer and the dissolved/molten polymer may be used in combination to form the droplets 2 and emulsify the droplets 2. When a biodegradable resin is selected as a polymer serving as a core of the composite particles, the composite particles are obtained as composite particles composed of a biodegradable resin as an inner core and CNF as an outer shell. Thus, the composite particles can be provided as composite particles that contain a biodegradable material and are highly environmentally-friendly.

### (Third step)

The third step is a step of solidifying the polymerizable monomer droplet and/or polymer droplet 2 to obtain the composite particle 5 in which the polymer particle 3 is coated with the fine cellulose 1.

The method of solidifying the polymerizable monomer droplet is not particularly limited, and may be appropriately selected according to the types of polymerizable monomer and polymerization initiator used, and may be, for example, suspension polymerization.

The specific method of the suspension polymerization is not particularly limited, and a known method may be used. For example, the suspension polymerization may be performed by heating while stirring the stabilized O/W emulsion produced at the second step in which the monomer droplets containing the polymerization initiator are coated with the fine cellulose 1. The stirring method is not particularly limited, and a known method may be used. Specifically, a dispersion device or a stirring bar may be used. Alternatively, the O/W emulsion may be only heated without being stirred. The temperature conditions for heating the O/W emulsion may be appropriately set according to the types of polymerizable monomer and polymerization initiator, but the temperature is preferably in the range of 20°C or more and 150°C or less. If the temperature is less than 20°C, the reaction rate of polymerization is reduced, which is not preferable. If the temperature is more than 150°C, the fine cellulose 1 may be modified, which is not preferable. The time for the polymerization reaction may be appropriately set according to the types of polymerizable monomer and polymerization initiator, but is typically in the range of approximately 1 to 24 hours. The polymerization reaction may be performed by ultraviolet irradiation treatment, i.e., using a type of electromagnetic waves. Other than electromagnetic waves, a particle beam such as an electron beam may be used.

The method of solidifying the polymer droplets is not particularly limited. For example, in the case where dissolved polymer droplets obtained by using a solvent are used, after the formation of an O/W emulsion in the CNF dispersion liquid, as described above, the solvent having low solubility in water is dispersed and transferred to an aqueous phase over time, so that the dissolved polymer is deposited and solidified as particles. For example, in the case where molten polymer droplets obtained by liquefying a polymer by heat are used, after the formation of an O/W emulsion in the CNF dispersion liquid, the emulsion is cooled, so that the molten polymer droplets are solidified as particles.

Through the steps described above, the spherical composite particles 5 are produced in which the polymer particles 3 are coated with the fine cellulose 1.

Immediately after the solidification treatment of the droplets 2 is completed, the dispersion liquid of the composite particles 5 contains both a large amount of water and the fine cellulose 1 that is released and does not contribute to the formation of the coating layer of the composite particles 5. Thus, the produced composite particles 5 need to be recovered and purified. The recovery and purification are preferably performed by centrifugal separation washing or filtration washing. The centrifugal separation washing may be performed by a known method. Specifically, an operation is repeated in which the composite particles 5 are precipitated by centrifugal separation and the supernatant liquid is removed, and the composite particles 5 are redispersed in a mixed solvent of water and methanol. Then, from a precipitate finally obtained by the centrifugal separation, the residual solvent is removed to recover the composite particles 5. The filtration washing may also be performed by a known method. For example, suction filtration with water and methanol is repeatedly performed by using a PTFE membrane filter with a pore diameter of 0.1 µm. Then, from a paste that finally remains on the membrane filter, the residual solvent is removed to recover the composite particles 5.

The method of removing the residual solvent is not particularly limited, and may be performed by air drying or simple heat drying using an oven or the like. The dry solid containing the composite particles 5 obtained in this manner is not formed into a film or an aggregate and is obtained as a fine-grained powder as described above.

The form of the skin application composition of the present invention is not particularly limited as long as the skin application composition is a composition containing the composite particles 5.

For example, the skin application composition can be provided as a dispersion liquid obtained by dispersing the composite particles 5 in an appropriate dispersion medium. The dispersion liquid may contain an additional component other than the composite particles 5. Examples of the additional component include an organometallic compound such as alkoxysilane or a hydrolysate thereof, an inorganic layered compound, an inorganic needle-like mineral, an antifoaming agent, inorganic particles, organic particles, a lubricant, an antioxidant, an antistatic agent, an ultraviolet absorber, a stabilizer, a magnetic powder, an orientation accelerator, a plasticizer, a crosslinking agent, a magnetic material, a pharmaceutical product, an agricultural chemical, a fragrance, an adhesive, an enzyme, a pigment, a dye, a deodorant, a metal, a metal oxide, and an inorganic acid.

For example, the composite particles can be provided as a fine-grained powder that contains no solvent and is intended to be used for a material applied to the skin. Specifically, the composite particles can be provided as a composition for a powder foundation for cosmetics. The powder may contain an additional component other than the composite particles 5. Examples of the additional component include an organometallic compound such as alkoxysilane or a hydrolysate thereof, an inorganic layered compound, an inorganic needle-like mineral, an antifoaming agent, inorganic particles, organic particles, a lubricant, an antioxidant, an antistatic agent, an ultraviolet absorber, a stabilizer, a magnetic powder, an orientation accelerator, a plasticizer, a crosslinking agent, a magnetic material, a pharmaceutical product, an agricultural chemical, a fragrance, an adhesive, an enzyme, a pigment, a dye, a deodorant, a metal, a metal oxide, and an inorganic acid.

The skin application composition of the present invention can be used not only as a component of a product applied to the skin but also as a component of a product used in the oral cavity such as toothpaste and a hair care product used for hair.

### (Advantageous effects of the present embodiment)

According to the skin application composition containing the composite particles 5 of the present embodiment, it is possible to provide a skin application composition containing novel composite particles, having high biocompatibility and good dispersion stability which are derived from the fine cellulose 1 on the surface thereof, with the good dispersion stability allowing the composite particles 5 to be prevented from aggregating in a solvent.

The dry solid containing the composite particles 5 according to the present embodiment is obtained as a fine-grained powder with no aggregation of the particles. Thus, the composite particles 5 obtained as a dry powder are easily redispersed in a solvent, and after the redispersion, the dispersion liquid also exhibits dispersion stability derived from the coating layer of the CNF 1 bonded to the surface of the composite particles 5. Therefore, it is possible to provide a skin application composition containing novel composite particles that utilizes the characteristics of the CNF and solves the excessive solvent problem.

One embodiment of the present invention has been described in detail above with reference to the drawings. However, the specific configuration should not be limited to this embodiment, but should include design changes within the scope not departing from the spirit of the present invention. Furthermore, the components described in the second embodiment and the modifications described above can be combined as appropriate.

### [Examples]

According to the first embodiment, the present invention will be described in detail below on the basis of examples, but the technical scope of the present invention is not limited to these examples. In the following examples, "%" indicates % by mass (w/w%) unless otherwise noted.

### <Example 1>

### (First step: Step of obtaining cellulose nanofiber dispersion liquid)

### (TEMPO oxidation of wood cellulose)

First, 70 g of softwood kraft pulp was suspended in 3500 g of distilled water, followed by adding a solution obtained by dissolving 0.7 g of TEMPO and 7 g of sodium bromide in 350 g of distilled water, followed by cooling to 20°C. To the resultant solution, 450 g of sodium hypochlorite aqueous solution with a concentration of 2 mol/L and a density of 1.15 g/mL was added dropwise to start an oxidation reaction. The temperature in the system was kept constant at 20°C. The pH during the reaction was maintained at pH 10 by adding 0.5 N sodium hydroxide aqueous solution according to the reduction of the pH. When the sum of the amount of added sodium hydroxide reached 3.50 mmol/g with respect to the weight of cellulose, approximately 100 mL of ethanol was added to stop the reaction. Then, filtration washing with distilled water was repeated by using a glass filter to obtain oxidized pulp.

### (Measurement of the amount of carboxyl groups of oxidized pulp)

A solid content weight of 0.1 g of oxidized pulp and reoxidized pulp obtained by the TEMPO oxidation was weighed and dispersed in water at a concentration of 1%, followed by adding hydrochloric acid so that the pH became 2.5. Then, the quantity of carboxyl groups (mmol/g) was determined by conductometric titration using 0.5 M sodium hydroxide aqueous solution. The result showed that the amount of carboxyl groups was 1.6 mmol/g.

### (Defibrating treatment of oxidized pulp)

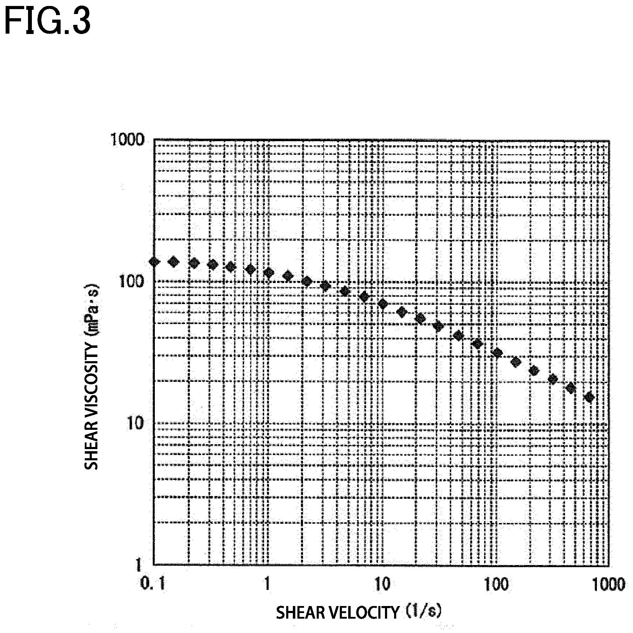

First, 1 g of oxidized pulp obtained by the TEMPO oxidation was dispersed in 99 g of distilled water, and finely ground by using a blender for 30 minutes, thereby obtaining a CSNF water dispersion liquid having a CSNF concentration of 1%. The CSNF dispersion liquid was placed in a quartz cell with an optical path length of 1 cm to measure a transmission spectrum by using a spectrophotometer ("UV-3600" manufactured by Shimadzu Corporation). Fig. 2 shows the measurement results. As is clear from Fig. 2, the CSNF water dispersion liquid exhibited high transparency. Furthermore, the CSNF contained in the CSNF water dispersion liquid had a number average minor axis diameter of 3 nm and a number average major axis diameter of 1110 nm. Fig. 3 shows the result of steady-state viscoelasticity measurement using a rheometer. As is clear from Fig. 3, the CSNF dispersion liquid exhibited thixotropic properties.

### (Second step: Step of producing O/W emulsion)

Next, to 1 g of cellulose acetate butyrate (trade name: CAB-551-0.2, hereinafter also referred to as CAB), which was a biodegradable compound, and 9 g of divinylbenzene (hereinafter also referred to as DVB), which was a polymerizable monomer, 1 g of 2,2-azobis-2,4-dimethylvaleronitrile (hereinafter also referred to as ADVN), which was a polymerization initiator, was dissolved. When the total amount of mixed solution of CAB, DVB, and ADVN was added to 40 g of CSNF dispersion liquid at a CSNF concentration of 1%, the mixed solution of CAB, DVB, and ADVN and the CSNF dispersion liquid was separated into two layers each of which had high transparency.

Next, a shaft of an ultrasonic homogenizer was inserted from a liquid surface of an upper layer of the mixture solution separated in two layers to perform ultrasonic homogenizer treatment for 3 minutes under conditions of a frequency of 24 kHz and an output of 400 W. The mixture solution after the ultrasonic homogenizer treatment had an opaque emulsified liquid appearance. When a drop of the mixture solution was placed on a glass slide and covered with a cover glass and then observed by using an optical microscope, it was confirmed that numerous emulsion droplets had been produced with a size of approximately one to several micrometers, and dispersed and stabilized as an O/W emulsion.

### (Third step: Step of obtaining composite particles 5 coated with CNF by polymerization reaction)

The O/W emulsion dispersion liquid was placed in hot water at 70°C by using a water bath and treated while being stirred with a stirring bar for 8 hours to cause a polymerization reaction. After the 8-hour treatment, the dispersion liquid was cooled to room temperature. The dispersion liquid showed no change in appearance before and after the polymerization reaction. The obtained dispersion liquid was centrifuged at a centrifugal force of 75,000 g (g represents gravitational acceleration) for 5 minutes to obtain a precipitate. The precipitate was recovered by removing the supernatant liquid by decantation, followed by repeatedly washing with pure water and methanol by using a PTFE membrane filter with a pore diameter of 0.1 µm. When the purified and recovered material obtained in this manner was redispersed at a concentration of 1% and the particle size of the material was evaluated by using a particle size distribution analyzer (NANOTRAC UPA-EX150, Nikkiso Co., Ltd.), the average particle size was 2.1 µm. Next, the purified and recovered material was air dried, followed by vacuum drying treatment at a room temperature of 25°C for 24 hours, thereby obtaining a fine-grained dry powder (composite particles 5).

### (Observation of shape using scanning electron microscope)

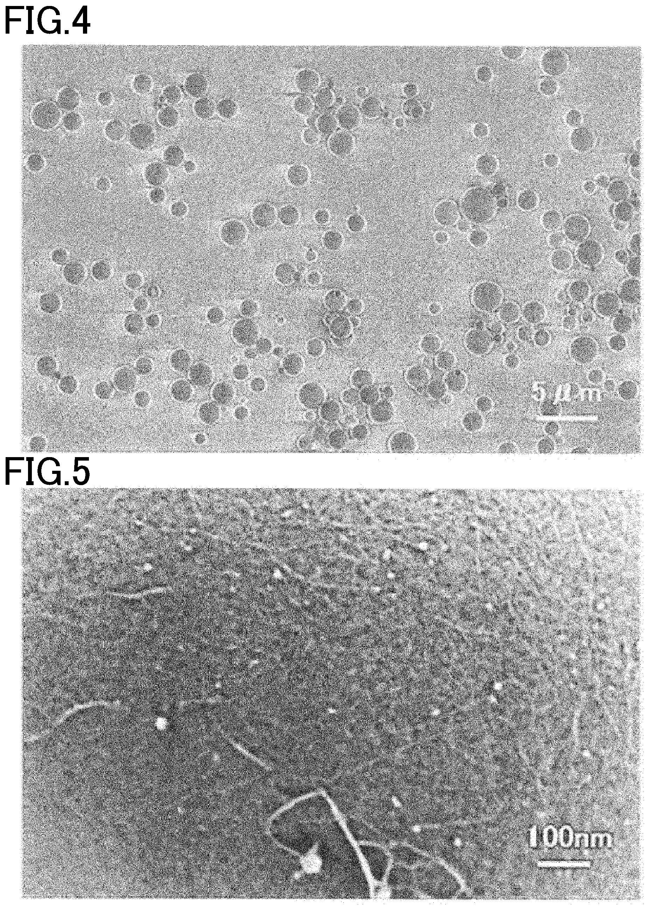

Figs. 4 and 5 show the results of observation of the obtained dry powder using a scanning electron microscope. As is clear from Fig. 4, since the polymerization reaction was performed by using the O/W emulsion droplets as a template, the formation of numerous spherical composite particles 5 derived from the shape of the emulsion droplets was confirmed. Furthermore, as shown in Fig. 5, it was confirmed that the surface of the composite particles 5 was uniformly coated with CNF having a width of several nanometers. Despite the repeated washing by filtration washing, the surface of the composite particles 5 was evenly and uniformly coated with the fine cellulose 1. This showed that in the composite particles 5 of the present embodiment, there was a possibility that a monomer (compound particle 3) inside the composite particle 5 and the CNF which was the fine cellulose 1 were bonded to each other, and the compound particles 3 and the fine cellulose 1 were inseparable.

### (Evaluation of redispersibility)

When the dry powder of the composite particles 5 was added to pure water at a concentration of 1% and redispersed with a stirring bar, the dry powder was easily redispersed and no aggregation was observed. When the particle size of the composite particles 5 was evaluated by using the particle size distribution analyzer, the average particle size was 2.1 µm, which was the same size as before drying, and in the data obtained by using the particle size distribution analyzer, no signal indicating aggregation was observed.

Thus, the results showed that even though the surface of the composite particles 5 was coated with the CNF, when dried, the composite particles 5 were not formed into a film, and were obtained as a powder and had good redispersibility.

### <Example 2>

The composite particles 5 according to Example 2 were produced under the same conditions as in Example 1, except that polybutylene succinate (trade name: BioPBS, Mitsubishi Chemical, hereinafter also referred to as PBS) was used instead of CBA and diethylene glycol diacrylate (trade name: FA-222A, Hitachi Chemical, hereinafter also referred to as FA-222A) was used instead of DVB. Then, various evaluations were performed in the same manner as in Example 1.

### <Example 3>

The composite particles 5 according to Example 3 were produced under the same conditions as in Example 1, except that a carboxymethylated CNF dispersion liquid was used. The carboxymethylated CNF dispersion liquid was obtained by, instead of the TEMPO oxidation, carboxymethylation according to Patent Literature 2 listed in the Citation List. Then, various evaluations were performed in the same manner as in Example 1.

### <Example 4>

The composite particles 5 according to Example 4 were produced under the same conditions as in Example 1, except that a phosphoric acid esterified CNF dispersion liquid was used. The phosphoric acid esterified CNF dispersion liquid was obtained by, instead of the TEMPO oxidation, phosphoric acid esterification according to Non Patent Literature 1 listed in the Citation List. Then, various evaluations were performed in the same manner as in Example 1.

### <Example 5>

As the biodegradable compound, 6 g of polylactic acid (trade name: TERRAMAC TE4000, hereinafter referred to as PLA) was dissolved in 4 g of dichloromethane (boiling point: 39.6°C). The total amount of mixed solution of polylactic acid and dichloromethane was added to 40 g of CSNF dispersion liquid used in Example 1.

Next, the mixture solution was subjected to ultrasonic homogenizer treatment for 3 minutes under conditions of a frequency of 24 kHz and an output of 400 W as in Example 1 to obtain an O/W emulsion dispersion liquid.

Next, the O/W emulsion dispersion liquid was placed in hot water at 70°C by using a water bath and treated while being stirred with a stirring bar for 8 hours to volatilize the dichloromethane. After the 8-hour treatment, the dispersion liquid was treated as in Example 1 to obtain a dry powder (composite particles 5) according to Example 5.

### <Example 6>

The composite particles 5 according to Example 6 were produced under the same conditions as in Example 5, except that polycaprolactone (trade name: TONE, UCC, hereinafter also referred to as PCL) was used instead of polylactic acid, and chloroform was used instead of dichloromethane. Then, various evaluations were performed in the same manner as in Example 5.

### <Example 7>

As the biodegradable compound, 10 g of stearic acid (melting point: 69.6°C) was added to 40 g of CSNF dispersion liquid used in Example 1.

Next, the mixture solution was heated to 80°C by using a water bath to melt the stearic acid, followed by ultrasonic homogenizer treatment for 3 minutes under conditions of a frequency of 24 kHz and an output of 400 W as in Example 1 to obtain an O/W emulsion dispersion liquid.

Next, the O/W emulsion dispersion liquid was cooled to room temperature.

Finally, the dispersion liquid was treated as in Example 1 to obtain a dry powder (composite particles 5) according to Example 7.

### <Example 8>

The composite particles 5 according to Example 8 were produced under the same conditions as in Example 7, except that paraffin wax (trade name: Paraffin wax 135°F, melting point: 57.2°C, Yamakei Sangyo, hereinafter also referred to as Paraffin) was used instead of stearic acid. Then, various evaluations were performed in the same manner as in Example 7.

### <Comparative Example 1>

Production of composite particles according to Comparative Example 1 was attempted under the same conditions as in Example 1, except that pure water was used instead of the CSNF dispersion liquid.

### <Comparative Example 2>

Production of composite particles according to Comparative Example 2 was attempted under the same conditions as in Example 1, except that a carboxymethyl cellulose (hereinafter also referred to as CMC) aqueous solution was used instead of the CSNF dispersion liquid.

### <Comparative Example 3>

Production of composite particles according to Comparative Example 3 was attempted under the same conditions as in Example 1, except that a dispersion liquid obtained by dispersing not the composite particles of the present invention but commercially available polylactic acid fine particles (trade name: Toraypearl PLA, Toray) at a concentration of 1% was used.

### <Comparative Example 4>

Production of composite particles according to Comparative Example 4 was attempted under the same conditions as in Example 5, except that xylene (boiling point: 144°C) was used instead of dichloromethane.

### <Comparative Example 5>

Production of composite particles according to Comparative Example 5 was attempted under the same conditions as in Example 7, except that oleic acid (melting point: 13.4°C) was used instead of stearic acid.

### <Comparative Example 6>

Production of composite particles according to Comparative Example 6 was attempted under the same conditions as in Example 7, except that amide wax (trade name: ITOHWAX-J630, melting point: 135°C, Yamakei Sangyo, hereinafter referred to as ITOWAX) was used instead of stearic acid.

Table 1 shows the evaluation results obtained by using Examples and Comparative Examples described above.

**[Table 1]**

| | Emulsion stabilizer | Contents | Result of second step | Result of third step | Redispersibility | Biodegradability |
|---|---|---|---|---|---|---|
| Example 1 | TEMPO-oxidized CNF | CAB/CVB/ADVN | Good | Good | Good | Good |
| Example 2 | TEMPO-oxidized CNF | PBS/FA-222A/ADVN | Good | Good | Good | Good |
| Example 3 | Carboxymethylated CNF | CAB/CVB/ADVN | Good | Good | Good | Good |
| Example 4 | Phosphoric acid esterified CNF | CAB/CVB/ADVN | Good | Good | Good | Good |
| Example 5 | TEMPO-oxidized CNF | PLA/Dichloromethane | Good | Good | Good | Good |
| Example 6 | TEMPO-oxidized CNF | PCL/Chloroform | Good | Good | Good | Good |
| Example 7 | TEMPO-oxidized CNF | Stearic acid | Good | Good | Good | Good |
| Example 8 | TEMPO-oxidized CNF | Paraffin | Good | Good | Good | Good |
| Comparative Example 1 | None | CAB/CVB/ADVN | Poor | | | |
| Comparative Example 2 | CMC | CAB/CVB/ADVN | Good | Poor | | |
| Comparative Example 3 | TEMPO-oxidized CNF | Toraypearl PLA | Good | Poor | | |
| Comparative Example 4 | TEMPO-oxidized CNF | PLA/Xylene | Good | Poor | | |
| Comparative Example 5 | TEMPO-oxidized CNF | Oleic acid | Good | Poor | | |
| Comparative Example 6 | TEMPO-oxidized CNF | ITOWAX | Poor | | | |

In Table 1, the emulsion stabilizer indicates an additive used to stabilize the O/W emulsion at the second step, and is, for example, the fine cellulose 1 of the present embodiment.

### [Evaluation criteria]

In Table 1, the result of the second step was determined as follows.
Good: O/W emulsion was formed.
Poor: No O/W emulsion was formed.

The result of the third step was determined as follows.
Good: Spherical particles were produced by using the emulsion at the third step as a template.
Poor: The above particles were not produced.

Redispersibility was determined as follows.
Good: The obtained composite particles were dispersed in the solvent (water) with no aggregation.
Poor: The obtained composite particles were aggregated instead of being dispersed.

Biodegradability was evaluated on the basis of Japanese Industrial Standards (JIS) "JIS K6950: 2000 Determination of the ultimate aerobic biodegradability of plastic materials in an aqueous medium - Method by measuring the oxygen demand in a closed respirometer". The composite particles 5 and activated sludge were added to an inorganic salt medium at 100 mg/L and 30 mg/L, respectively. Then, oxygen consumption was measured to calculate oxygen consumption biochemical oxygen demand (BOD; the mass concentration of dissolved oxygen consumed by aerobic biological oxidation for a chemical substance or an organic material in water under specific conditions).

As a control, an inorganic salt medium containing no composite particles 5 was used. The amount of oxygen (theoretical oxygen demand, ThOD) required for all the composite particles 5 to be converted into water and carbon dioxide gas was calculated by a polymer composition formula. The degree of biodegradation was calculated as biochemical oxygen demand with respect to theoretical oxygen demand (the degree of biodegradation = BOD/ThOD × 100). Biodegradability was determined according to the following criteria.
Good: The degree of biodegradation after 28 days from the start of the test was 80% or more.
Poor: The degree of biodegradation after 28 days from the start of the test was less than 80%.

A diagonal line in the cells for Comparative Examples in Table 1 indicates that at a point during the step, it became impossible to continue the step, and the step was stopped at that point.

As is clear from the evaluation results of Examples 1 to 8 in Table 1, it was confirmed that the biodegradable composite particles 5 were produced regardless of the type of fine cellulose 1 (TEMPO-oxidized CNF, carboxymethylated CNF, phosphoric acid esterified CNF), or the type of biodegradable compound or the type of polymerizable monomer, and thus the composite particles 5 are effective to solve the problem to be solved by the present invention.

On the other hand, in Comparative Example 1, the second step was unable to be completed. Specifically, even when ultrasonic homogenizer treatment was performed, the monomer layer and the CNF dispersion liquid layer were maintained to be separated in two layers, and no O/W emulsion was produced.

In Comparative Example 2, an O/W emulsion was formed at the second step. This is presumably because CMC exhibited amphipathic properties as with the fine cellulose, and thus CMC functioned as an emulsion stabilizer. However, when a polymerization reaction was performed at the subsequent third step, the emulsion collapsed, and no composite particles were produced by using the O/W emulsion as a template. The reason is uncertain, but the emulsion collapsed during the polymerization reaction presumably because CMC, which was water soluble, was highly likely to be brittle as a coating layer for maintaining the emulsion form during the polymerization reaction.

In Comparative Example 3, in which the commercially available PLA fine particles that had a similar configuration and were not coated with CNF were used, the surface of the fine particles coated with the fine cellulose was not observed by shape observation using the SEM.

In Comparative Example 4, an O/W emulsion was formed at the second step. However, during the removal of xylene at the third step, due to water used for the CNF dispersion liquid, the CNF dispersion liquid was unable to be heated to the boiling point of xylene or more. Thus, the biodegradable compound droplets 2 were not solidified, and no composite particles were obtained.

In Comparative Example 5, an O/W emulsion was formed at the second step. However, even when the O/W emulsion was cooled to room temperature at the third step, the oleic acid was not solidified. Thus, the biodegradable compound droplets 2 were not solidified, and no composite particles were obtained.

In Comparative Example 6, at the second step, the CNF dispersion liquid was unable to be heated to a temperature higher than the boiling point of water used for the CNF dispersion liquid. Accordingly, the ITOWAX was not melted, and thus no O/W emulsion was formed.

According to the second embodiment, the present invention will be described in detail below on the basis of examples, but the technical scope of the present invention is not limited to these examples. In the following examples, "%" indicates % by mass (w/w%) unless otherwise noted.

### <Example 9>

### (First step: Step of obtaining cellulose nanofiber dispersion liquid)

### (TEMPO oxidation of wood cellulose)

First, 70 g of softwood kraft pulp was suspended in 3500 g of distilled water, followed by adding a solution obtained by dissolving 0.7 g of TEMPO and 7 g of sodium bromide in 350 g of distilled water, followed by cooling to 20°C. To the resultant solution, 450 g of sodium hypochlorite aqueous solution with a concentration of 2 mol/L and a density of 1.15 g/mL was added dropwise to start an oxidation reaction. The temperature in the system was kept constant at 20°C. The pH during the reaction was maintained at pH 10 by adding 0.5 N sodium hydroxide aqueous solution according to the reduction of the pH. When the sum of the amount of added sodium hydroxide reached 3.50 mmol/g with respect to the weight of cellulose, approximately 100 mL of ethanol was added to stop the reaction. Then, filtration washing with distilled water was repeated by using a glass filter to obtain oxidized pulp.

### (Measurement of the amount of carboxyl groups of oxidized pulp)

A solid content weight of 0.1 g of oxidized pulp and reoxidized pulp obtained by the TEMPO oxidation was weighed and dispersed in water at a concentration of 1%, followed by adding hydrochloric acid so that the pH became 2.5. Then, the quantity of carboxyl groups (mmol/g) was determined by conductometric titration using 0.5 M sodium hydroxide aqueous solution. The result showed that the amount of carboxyl groups was 1.6 mmol/g.

### (Defibrating treatment of oxidized pulp)

First, 1 g of oxidized pulp obtained by the TEMPO oxidation was dispersed in 99 g of distilled water, and finely ground by using a blender for 30 minutes, thereby obtaining a CSNF water dispersion liquid having a CSNF concentration of 1%. The CSNF dispersion liquid was placed in a quartz cell with an optical path length of 1 cm to measure a transmission spectrum by using a spectrophotometer ("UV-3600" manufactured by Shimadzu Corporation). Fig. 2 shows the measurement results. As is clear from Fig. 2, the CSNF water dispersion liquid exhibited high transparency. Furthermore, the CSNF contained in the CSNF water dispersion liquid had a number average minor axis diameter of 3 nm and a number average major axis diameter of 1110 nm. Fig. 3 shows the result of steady-state viscoelasticity measurement using a rheometer. As is clear from Fig. 3, the CSNF dispersion liquid exhibited thixotropic properties.

### (Second step: Step of producing O/W emulsion)

First, 6 g of polylactic acid (trade name: TERRAMAC TE4000, hereinafter referred to as PLA) was dissolved in 4 g of dichloromethane (boiling point: 39.6°C), followed by adding the total amount of mixed solution of polylactic acid and dichloromethane to 40 g of CSNF dispersion liquid.

The mixture solution was subjected to ultrasonic homogenizer treatment for 3 minutes under conditions of a frequency of 24 kHz and an output of 400 W to obtain an O/W emulsion dispersion liquid.

### (Third step: Step of solidifying droplets in emulsion to obtain composite particles 5)

The O/W emulsion dispersion liquid was placed in hot water at 70°C by using a water bath and was stirred with a stirring bar for 8 hours to volatilize dichloromethane. After the 8-hour treatment, the dispersion liquid was centrifuged at a centrifugal force of 75,000 g for 5 minutes to obtain a precipitate. The precipitate was recovered by removing the supernatant liquid by decantation, followed by repeatedly washing with pure water and methanol by using a PTFE membrane filter with a pore diameter of 0.1 µm. When the purified and recovered material obtained in this manner was redispersed at a concentration of 1% and the particle size of the material was evaluated by using a particle size distribution analyzer (NANOTRAC UPA-EX150, Nikkiso Co., Ltd.), the average particle size was 2.1 µm. Next, the purified and recovered material was air dried, followed by vacuum drying treatment at a room temperature of 25°C for 24 hours, thereby obtaining a fine-grained dry powder (composite particles 5).

### (Observation of shape using scanning electron microscope)

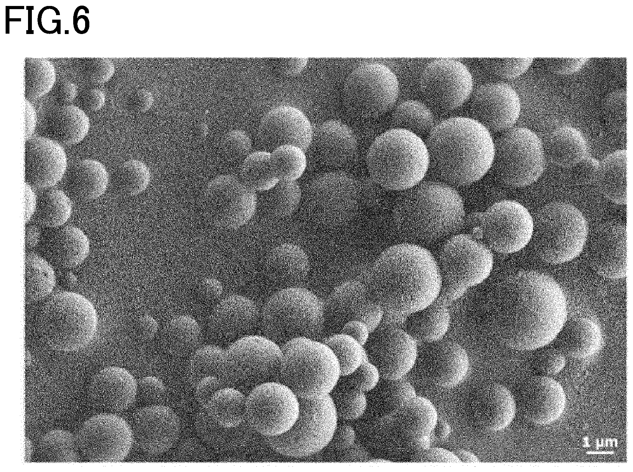

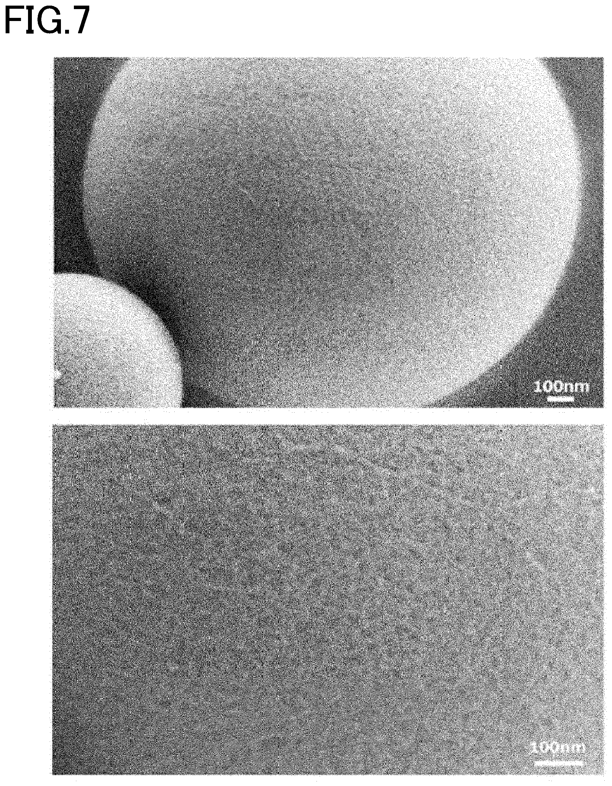

Figs. 6 and 7 show the results of observation of the obtained dry powder using a scanning electron microscope. As is clear from Fig. 6, since the polymerization reaction was performed by using the O/W emulsion droplets as a template, the formation of numerous spherical composite particles 5 derived from the shape of the emulsion droplets was confirmed. Furthermore, as shown in Fig. 7, it was confirmed that the surface of the composite particles 5 was uniformly coated with CNF having a width of several nanometers. Despite repeated washing by filtration washing, the surface of the composite particles 5 was evenly and uniformly coated with the CNF 1. This showed that in the composite particles 5 of the present invention, a monomer inside the composite particle and the CNF were bonded to each other and were inseparable.

### (Evaluation of redispersibility)

When the dry powder of the composite particles 5 was added to pure water at a concentration of 1% and redispersed with a stirring bar, the dry powder was easily redispersed and no aggregation was observed. When the particle size of the composite particles 5 was evaluated by using the particle size distribution analyzer, the average particle size was 2.1 µm, and in the data obtained by the particle size distribution analyzer, no signal indicating aggregation was observed. Thus, the results showed that even though the surface of the composite particles 5 was coated with the CNF, when dried, the composite particles 5 were not formed into a film, and were obtained as a powder and had good redispersibility.

### (Evaluation of moisture retention properties and feeling in use)

In a room at a temperature of 20°C and a humidity of 40% RH, the dry powder (composite particles 5) was applied to an inner part of the lower arm of a subject (woman in her thirties). Specifically, 0.1 g of dry powder was applied to the skin and spread with a finger into a circle with a diameter of approximately 5 cm. When 30 minutes had elapsed after the application, at the part at which the dry powder was applied, the amount of skin moisture was measured by using a Moisture checker MY-707S (Scalar Corporation). Furthermore, the feeling of the dry powder when applied to the skin was determined as follows.
Good: Highly compatible with the skin and little or no irritation to the skin.
Poor: Poorly compatible with the skin and irritating to the skin.

### <Example 10>

The composite particles 5 were produced under the same conditions as in Example 9, except that polycaprolactone (trade name: TONE, UCC, hereinafter also referred to as PCL) was used instead of polylactic acid, and chloroform was used instead of dichloromethane. Then, various evaluations were performed in the same manner as in Example 9.

### <Example 11>

The composite particles 5 were produced under the same conditions as in Example 9, except that the second step and the third step were changed as described later. Then, various evaluations were performed in the same manner as in Example 9.

### (Second step: Step of producing O/W emulsion)

First, 10 g of stearic acid (melting point: 69.6°C) was added to 40 g of CSNF dispersion liquid. Next, the mixture solution was heated to 80°C by using a water bath to melt the stearic acid, followed by ultrasonic homogenizer treatment for 3 minutes under conditions of a frequency of 24 kHz and an output of 400 W as in Example 9 to obtain an O/W emulsion dispersion liquid.

### (Third step: Step of solidifying droplets in emulsion to obtain composite particles 5)

The O/W emulsion dispersion liquid was cooled to room temperature to solidify the molten stearic acid droplets 2. The dispersion liquid was treated as in Example 9 to obtain a dry powder (composite particles 5).

### <Comparative Example 7>

Instead of using the composite particles 5 of the present invention, a CSNF powder was used. The CSNF powder was obtained by freeze-drying a CSNF water dispersion liquid to form dried CSNF, followed by freeze-grinding treatment. Then, various evaluations were performed in the same manner as in Example 9. When the shape of the CSNF powder was observed by using an optical microscope, it was found that the obtained particles were deformed particles, and greatly varied in particle size between approximately 10 to 100 µm.

### <Comparative Example 8>

Instead of using the composite particles 5 of the present invention, commercially available styrene-divinylbenzene copolymer microbeads (particle size: 4.5 µm, Techno Chemical) were used. Then, various evaluations were performed in the same manner as in Example 9.

Table 2 shows the evaluation results obtained by using Examples and Comparative Examples described above.

**[Table 2]**

| | Emulsion stabilizer | Composition of droplets 2 | Redispersibility | Amount (%) of skin moisture | Feeling in use |
|---|---|---|---|---|---|
| Example 9 | TEMPO-oxidized CNF | PLA/Dichloromethane | Good | 67 | Good |
| Example 10 | TEMPO-oxidized CNF | PCL/Chloroform | Good | 66 | Good |
| Example 11 | TEMPO-oxidized CNF | Stearic acid | Good | 63 | Good |
| Comparative Example 7 | Dried and ground CSNF used | | Poor | 55 | Poor |
| Comparative Example 8 | Commercially available microbeads used | | Poor | 46 | Good |

In Table 2, the emulsion stabilizer indicates an additive used to stabilize the O/W emulsion at the second step, and is for example, the fine cellulose 1 of the present invention.

As is clear from the evaluation results of Examples 9 to 11 in Table 2, regardless of the composition of the droplets 2, when the powder was applied to the skin, the amount of skin moisture was 60% or more and good moisture retention properties were exhibited. This is presumably because, due to high immiscibility between the skin and the fine-grained composite particles having the surface to which the hydrophilic CNF was bonded, higher moisture retention properties were exhibited. Furthermore, good redispersibility was exhibited. This is presumably because, due to the osmotic pressure effect derived from the anionic functional group of the TEMPO-oxidized CNF on the surface of the composite particles, water was more likely to enter between the composite particles. Furthermore, since the powder was a fine-grained powder, the powder was highly compatible with the skin and provided a very good feeling when in use. The results showed that the powder containing the composite particles 5 of the present invention is suitable as a skin application composition, considering that, for example, a powder foundation applied to the skin is easily dried.

On the other hand, in Comparative Example 7, possibly because the CNF were aggregated and keratinized by the freeze-drying treatment, the particles were difficult to be redispersed. Furthermore, due to the variation in the particle shape and the particle size, the powder was poorly compatible with the skin and caused an unsatisfactory feeling in use. Possibly due to the poor compatibility with the skin, as compared with Examples 9 to 11, poor moisture retention properties were exhibited.

In Comparative Example 8, in which the commercially available fine-grained beads that had a spherical shape and a uniform particle size were used, the powder was highly compatible with the skin and provided a good feeling in use. However, unlike in Examples 9 to 11, since the CNF having high hydrophilicity was not bonded to the surface of the commercially available beads, the commercially available beads did not exhibit moisture retention properties, and the amount of skin moisture was as low as 46%. As compared with Examples 9 to 11, due to lack of the TEMPO-oxidized CNF on the surface of the beads, poor redispersibility was observed.

As described above, it was confirmed that, as a skin application composition, the compositions containing the composite particles 5 of Examples 9 to 11 are effective to solve the problem to be solved by the present invention.

### [Industrial Applicability]

The composite particle of the present invention has advantageous effects in terms of industrial implementation such as contribution to cost reduction due to improvement in addition efficiency as an additive, improvement in efficiency of kneading with resin, improvement in transport efficiency, and prevention of spoilage. The composite particle of the present invention contains the biodegradable compound, which is the characteristic of the composite particle of the present invention, and the CNF with which the compound is coated is also a biodegradable material. Thus, the composite particle of the present invention is an environmentally-friendly material. By utilizing the characteristics of the fine cellulose on the particle surface of the composite particle and the polymer contained in the composite particle, the composite particle of the present invention is applicable to colorants, adsorbents, cosmetics pigments, sustained-release drugs, deodorants, antibacterial medical members, antibacterial articles for personal care products, packaging materials, dye-sensitized solar cells, photoelectric conversion materials, photothermal conversion materials, heat shielding materials, optical filters, Raman enhancing elements, image display elements, magnetic powders, catalyst supports, drug delivery systems, and the like.

The skin application composition containing the composite particle of the present invention allows simple removal of the solvent while maintaining the characteristics of the CNF, thereby achieving advantageous effects in terms of industrial implementation such as contribution to cost reduction due to improvement in addition efficiency and prevention of spoilage. By utilizing the characteristics of the fine cellulose on the particle surface of the composite particle, the functional material added in addition to the fine cellulose, and the polymer contained in the composite particle, the composite particle contained in the skin application composition of the present invention is applicable to colorants, adsorbents, cosmetics pigments, sustained-release drugs, deodorants, antibacterial members, packaging materials, catalyst supports, drug delivery systems, and the like for skin application products.

### [Reference Signs List]

1: Cellulose nanofiber (fine cellulose)
2: Biodegradable compound droplet (polymerizable monomer droplet and/or polymer droplet)
3: Biodegradable compound particle (polymer particle)
4: Dispersion liquid
5: Composite particle

## Claims

1. A composite particle comprising:
a particle containing at least one type of biodegradable compound; and
fine cellulose with which at least part of a surface of the particle containing the biodegradable compound is coated,
the particle containing the biodegradable compound and the fine cellulose being inseparable.

2. A method of producing a composite particle, comprising:
a first step of defibrating a cellulose raw material in a solvent to obtain a dispersion liquid of fine cellulose;
a second step of coating at least part of a surface of a droplet containing a biodegradable compound with the fine cellulose in the dispersion liquid to stabilize the droplet containing the biodegradable compound as an emulsion; and
a third step of, while at least part of the surface of the droplet containing the biodegradable compound is coated with the fine cellulose, solidifying the droplet containing the biodegradable compound to form a polymer particle so that at least part of a surface of the polymer particle is coated with the fine cellulose and the polymer particle and the fine cellulose are inseparable.

3. The method of producing a composite particle according to claim 2, wherein the second step is one of a step in which a mixture of the biodegradable compound and a polymerizable monomer containing an initiator is added to the dispersion liquid of the fine cellulose and emulsified, a step in which a solution obtained by dissolving the biodegradable compound in a solvent having low immiscibility with the dispersion liquid of the fine cellulose is added to the dispersion liquid of the fine cellulose and emulsified, and a step in which the biodegradable compound that is solid at normal temperature is added to the dispersion liquid of the fine cellulose and heated to a melting point of the biodegradable compound or more so that the biodegradable compound is melted and emulsified.

4. A dry powder comprising the composite particle according to claim 1 and having a solid content ratio of 80% or more.

5. A skin application composition comprising a composite particle that contains at least one type of polymer particle and has a coating layer that is composed of fine cellulose and provided on a surface of the polymer particle, the polymer particle and the fine cellulose being bonded to each other and inseparable.

6. The skin application composition according to claim 5, wherein the fine cellulose includes an anionic functional group on a crystal surface of the fine cellulose.

7. The skin application composition according to claim 5 or 6, wherein the polymer particle is obtained by polymerizing a monomer having a vinyl group.

8. The skin application composition according to any one of claims 5 to 7, wherein the polymer particle is obtained by polymerizing a monomer having a (meth)acrylic group.

9. The skin application composition according to any one of claims 5 to 8, wherein the polymer particle is obtained by polymerizing a polyfunctional monomer having two or more polymerizable functional groups.

10. The skin application composition according to claim 9, wherein at least one of the two or more polymerizable functional groups of the polyfunctional monomer is a vinyl group.

11. The skin application composition according to claim 10, wherein at least one of the two or more polymerizable functional groups of the polyfunctional monomer is a (meth)acrylic group.

12. The skin application composition according to claim 11, wherein the polyfunctional monomer is divinylbenzene.

13. The skin application composition according to claim 5 or 6, wherein the polymer particle is a biodegradable polymer.

14. The skin application composition according to claim 13, wherein the biodegradable polymer is polycaprolactone.

15. A method of producing a skin application composition, the method comprising:
a first step of defibrating a cellulose raw material in a solvent to obtain a dispersion liquid of fine cellulose;
a second step of coating a surface of a polymer droplet with the fine cellulose in the dispersion liquid to stabilize the polymer droplet as an emulsion; and
a third step of solidifying the polymer droplet to obtain a composite particle in which a polymer is coated with the fine cellulose.

16. A method of producing a skin application composition, the method comprising:
a first step of defibrating a cellulose raw material in a solvent to obtain a dispersion liquid of fine cellulose;
a second step of coating a surface of a polymerizable monomer and polymer droplet with the fine cellulose in the dispersion liquid to stabilize the polymerizable monomer and polymer droplet as an emulsion; and
a third step of solidifying the polymerizable monomer and polymer droplet to obtain a composite particle in which a polymer is coated with the fine cellulose.
